# EUROPÄISCHE PATENTSCHRIFT

(11) **EP 4 444 684 B1**
(45) Veröffentlichungstag und Bekanntmachung des Hinweises auf die Patenterteilung: **16.07.2025**
(21) Anmeldenummer: 22834562.5
(22) Anmeldetag: 08.12.2022
(51) Int. Cl.: C07C 5/48, C07C 7/04, C07C 7/00, C07C 51/25, C07C 9/06, C07C 11/04, C07C 53/08, C10G 9/36, C10G 51/06, C10G 70/02, C10G 70/04

(54) **VERFAHREN UND ANLAGE ZUR HERSTELLUNG EINES ODER MEHRERER KOHLENWASSERSTOFFE**
METHOD AND INSTALLATION FOR THE PRODUCTION OF ONE OR MORE HYDROCARBONS
PROCÉDÉ ET INSTALLATION DE PRODUCTION D'AU MOINS UN HYDROCARBURE

(30) Priorität: 08.12.2021 EP 21020623
(43) Veröffentlichungstag der Anmeldung: 16.10.2024
(73) Patentinhaber: Linde GmbH, 82049 Pullach (DE)
(72) Erfinder: KRACKER, Gunther, 83629 Weyarn (DE); ZELLHUBER, Mathieu, 82152 Planegg (DE); MCCRACKEN, Sean, 82178 Puchheim (DE); MEISWINKEL, Andreas, 83253 Rimsting (DE); SCHUBERT, Martin, 81375 München (DE); TOTA, Desislava, 81479 München (DE)
(86) Internationale Anmeldenummer: PCT/EP2022/084977
(87) Internationale Veröffentlichungsnummer: WO 2023/104963

(56) Entgegenhaltungen:
- WO-A1-2018/024650

## Beschreibung

Die Erfindung betrifft ein Verfahren und eine Anlage zur Herstellung eines oder mehrerer Kohlenwasserstoffe.

### Technischer Hintergrund

Das Steamcracken von Kohlenwasserstoffen (engl. Steam Cracking, im Deutschen auch als Dampfspalten bezeichnet) ist beispielsweise im Artikel "Ethylene" in Ullmann's Encyclopedia of Industrial Chemistry, Onlineausgabe, 15. April 2009, DOI: 10.1002/14356007.a10_045.pub2, beschrieben. Es wird vorwiegend zur Gewinnung von kurzkettigen Olefinen wie Ethylen und Propylen, Diolefinen wie Butadien, oder von Aromaten eingesetzt, ist jedoch nicht hierauf beschränkt.

Die oxidative Dehydrierung (engl. Oxidative Dehydrogenation, ODH) von Paraffinen mit zwei bis vier Kohlenstoffatomen (im Falle der oxidativen Dehydrierung von Ethan zu Ethylen auch als ODHE bzw. ODH-E bezeichnet) ist ebenfalls bekannt. Bei der oxidativen Dehydrierung werden die genannten Paraffine mit Sauerstoff unter anderem zu den jeweiligen Olefinen und zu Wasser umgesetzt. Sie stellt ein alternatives und in bestimmten Fällen vorteilhaftes Verfahren zur Herstellung von Olefinen dar.

Wie nachfolgend noch erläutert, sind beispielsweise aus der WO 2018/024650 A1, der WO 2014/134703 A1 und der CN 103086824 B auch Kombinationsverfahren aus Steamcracken und oxidativer Dehydrierung bekannt. Die vorliegende Erfindung stellt sich die Aufgabe, entsprechende Kombinationsverfahren zu verbessern.

### Offenbarung der Erfindung

Es wird ein Verfahren zur Herstellung eines oder mehrerer Kohlenwasserstoffe vorgeschlagen, bei dem ein erster Einsatzstrom unter Erhalt eines ersten Produktstroms einem Steamcracken und ein Ethan enthaltender zweiter Einsatzstrom unter Erhalt eines zweiten Produktstroms einer oxidativen Dehydrierung unterworfen werden, wobei unter Verwendung zumindest eines Teils des ersten Produktstroms und unter Verwendung zumindest eines Teils des zweiten Produktstroms ein Demethanisierungseinsatzstrom gebildet wird, der zumindest zum Teil einer Demethanisierung unterworfen wird, und bei dem bei der Bildung des Demethanisierungseinsatzstrom eine zumindest teilweise Sauerstoffentfernung vorgenommen wird. Zumindest ein Teil des ersten Produktstroms wird unter Erhalt einer leichteren Fraktion und einer schwereren Fraktion separat von dem zweiten Produktstrom einer Deethanisierung oder einer Depropanisierung unterworfen, wobei der Demethanisierungseinsatzstrom unter Vereinigung zumindest eines Teils der leichteren Fraktion und zumindest eines Teils des zweiten Produktstroms gebildet wird, und wobei die Sauerstoffentfernung stromab der Vereinigung vorgenommen wird.

Es wird vorgeschlagen, dass zumindest ein Teil des ersten Produktstroms ohne vorherige Acetylenhydrierung oder unter nur teilweiser Acetylenhydrierung der Vereinigung zugeführt wird, und dass die Sauerstoffentfernung eine Acetylenentfernung umfasst.

Ein besonderer Vorteil einer derartigen Vorgehensweise besteht insbesondere darin, dass eine separate Acetylenhydrierung im ersten Produktstrom des Steamcrackers auch vollständig entfallen kann, sofern die Sauerstoffentfernung derart durchgeführt wird, dass hierbei zugleich eine entsprechende spezifikationsgerechte Acetylenentfernung bewirkt wird (was i.d.R. der Fall ist). Entsprechende Apparate müssen daher nicht aufwendig bereitgestellt werden. Zu weiteren Vorteilen wird auf die ausführliche Beschreibung der Erfindung und ihrer Varianten unten verwiesen.

Insgesamt wird gemäß Ausgestaltungen der Erfindung eine optimierte Integration einer oxidativen Dehydrierung von Ethan und eines Steamcrackens geschaffen. Insbesondere ergibt sich der Vorteil einer gemeinsamen Nutzung bestimmter Verfahrensschritte bzw. Komponenten, insbesondere einer Demethanisierung und einer Trennung von Kohlenwasserstoffen mit zwei Kohlenstoffatomen voneinander (C2-Splitter). Beispielsweise besteht in Ausgestaltungen der Erfindung auch die Möglichkeit zur Kapazitätserweiterung eines Steamcrackers durch Nutzung der oxidativen Dehydrierung von Ethan, insbesondere für einen Ethanrecycle. Auch für reine oder überwiegende Flüssigeinsätze eingerichtete Steamcracker können damit derart erweitert werden, dass ein entsprechender Ethanrecycle genutzt werden kann. Durch eine oxidative Dehydrierung von Ethan kann zusätzlich Essigsäure in integrierten Komplexen bereitgestellt werden. Es ist insbesondere eine bedarfsgerechte Optimierung und Anpassung der Ethylenkapazität möglich. Ferner ist eine Reduktion des Frischwasserbedarfs als Einsatzstoff insbesondere für den Steamcracker durch Rückgewinnung von bei der oxidativen Dehydrierung von Ethan gebildetem zusätzlichem Wasser möglich. In der oxidativen Dehydrierung von Ethan wird deutlich mehr Wasser gebildet als dieser zugeführt wird. Dieses zusätzliche Wasser kann dann teilweise den Wassereinsatzstrom des Steamcracker zugeführt werden, wodurch sich weitere Umwelt- und Kostenvorteile für Frisch- und Abwasser ergeben.

In einem nicht erfindungsgemäßen Vergleichsbeispiel - nachfolgend insbesondere auch als "Variante 1" bezeichnet - ist vorgesehen, dass unter Vereinigung zumindest eines Teils des ersten Produktstroms und zumindest eines Teils des zweiten Produktstroms ohne vorherige Abtrennung gasförmiger Kohlenwasserstoffe ein Sammelstrom gebildet und zumindest zum Teil einer Kohlendioxidentfernung unterworfen wird, wobei der Demethanisierungseinsatzstrom unter Verwendung zumindest eines Teils eines der Kohlendioxidentfernung entnommenen Entnahmestroms gebildet wird. In dieser Ausgestaltung ist insbesondere eine gemeinsame Nutzung der Kohlendioxidentfernung und aller Komponenten stromab einer entsprechenden Kohlendioxidentfernung möglich. Jedoch können in diesem ersten Vergleichsbeispiel die Vorteile der stromab der Vereinigung erfolgenden Sauerstoffentfernung nicht erzielt werden.

In einem alternativen, nicht erfindungsgemäßen zweiten Vergleichsbeispiel und Ausgestaltungen der vorliegenden Erfindung - nachfolgend insbesondere auch als "Variante 2" und "Variante 3" bezeichnet - ist vorgesehen, dass zumindest ein Teil des ersten Produktstroms unter Erhalt einer leichteren Fraktion und einer schwereren Fraktion separat von dem zweiten Produktstrom einer Deethanisierung oder einer Depropanisierung unterworfen wird, wobei der Demethanisierungseinsatzstrom unter Vereinigung zumindest eines Teils der leichteren Fraktion und zumindest eines Teils des zweiten Produktstrom gebildet wird. In dieser Gruppe von Ausgestaltungen ergibt sich insbesondere eine Vereinfachung der Rohgasbehandlung der oxidativen Dehydrierung von Ethan vor einer entsprechenden Vereinigung, da Sauerstoff nicht extrem tief entfernt werden muss. Eine Abreicherung an Sauerstoff wird vorteilhafterweise jedoch bis auf einen Wert vorgenommen, der ausreichend niedrig ist, um auf eine druck- bzw. explosionsfeste Auslegung von nachfolgenden Anlagenteilen, wie z.B. in der Demethanisierung, verzichten zu können. Ein verbleibender Sauerstoffrestgehalt im Demethanisierungseinsatzstrom kann in der Demethanisierung entfernt werden. Hierdurch ergibt sich eine Minimierung des Ethylenverlustes stromauf insbesondere der Demethanisierung.

Generell soll hier unter dem Begriff "Rohgasbehandlung" eine Behandlung des Produktstroms aus der oxidativen Dehydrierung, oder eines hiervon abgeleiteten Stroms, verstanden werden, unabhängig davon, an welcher Stelle eines entsprechenden Prozesses sie stattfindet. Eine Rohgasbehandlung im hier verstandenen Sinn erfolgt jedoch immer vor der Vereinigung mit einem Strom aus dem Steamcracken.

Inden nicht erfindungsgemäßen Vergleichsbeispielen - nämlich insbesondere gemäß der bereits erwähnten "Variante 1" und gemäß der bereits erwähnten "Variante 2" - ist insbesondere vorgesehen, dass der zweite Produktstrom stromauf der Vereinigung einer Aufbereitung unterworfen wird, die die Sauerstoffentfernung umfasst. Die Sauerstoffentfernung braucht in dieser Ausgestaltung lediglich an die Anforderungen im Produktstrom der oxidativen Dehydrierung angepasst zu werden.

In derartigennicht erfindungsgemäßen Verfahrensvarianten - also insbesondere gemäß der bereits erwähnten "Variante 1" und gemäß der bereits erwähnten "Variante 2" - ist insbesondere vorgesehen, dass die Aufbereitung einen oder mehrere zusätzliche Schritte umfasst, der oder die aus einer Kondensatabtrennung, einer Vorverdichtung, einer Kohlendioxidentfernung und einer Trocknung ausgewählt sind. Auf diese Weise kann ein typischerweise deutlich höherer Kohlendioxidgehalt in dem Produktstrom der oxidativen Dehydrierung vorab bereits deutlich reduziert werden, um dann die nach der Vereinigung erfolgende (weitere) Kohlendioxidabtrennung in Form einer Feinreinigung vorzunehmen. Die Kondensatabtrennung ermöglicht insbesondere die oben erwähnte Gewinnung von Essigsäure als Wertprodukt und Wasser als Einsatzstoff.

Im Rahmen der vorliegenden Erfindung - nämlich insbesondere gemäß der bereits erwähnten "Variante 3" - ist vorgesehen, dass die Sauerstoffentfernung stromab der Vereinigung vorgenommen wird. Wie erwähnt, kann durch diese Maßnahmen bewirkt werden, dass eine separate Acetylenhydrierung im ersten Produktstrom des Steamcrackers auch vollständig entfallen kann, sofern die Sauerstoffentfernung derart durchgeführt wird, dass hierbei zugleich eine entsprechende spezifikationsgerechte Acetylenentfernung bewirkt wird (was i.d.R. der Fall ist). Die vorliegende Erfindung sieht dies vor, betrifft also ein Verfahren, bei dem zumindest ein Teil des ersten Produktstroms ohne vorherige Acetylenhydrierung oder unter nur teilweiser Acetylenhydrierung der Vereinigung zugeführt wird, und bei dem die Sauerstoffentfernung eine Acetylenentfernung umfasst.

Dies ist insbesondere gleichbedeutend damit, dass die leichtere Fraktion oder der Teil hiervon, der der Vereinigung unterworfen wird, einen Gehalt von beispielsweise 500 vol.ppm bis 30.000 vol.ppm (Millionstel Volumenanteile) Acetylen aufweist. Ein entsprechender Wert kann auch bei 2.000 vol.ppm bis 25.000 vol.ppm oder 3.000 vol.ppm bis 20.000 vol.ppm an Acetylen liegen, wobei der Gehalt an Acetylen auch eine Obergrenze von 15.000 vol.ppm erreichen kann.

Nach einer derartigen kombinierten Sauerstoff- und Acetylenentfernung ist insbesondere ein Gehalt von deutlich weniger als 1 vol.-ppm Acetylen anzustreben. Dies ist insbesondere deshalb der Fall, weil Acetylen später im Ethylen noch aufkonzentriert wird, wenn leichtere Komponenten und Ethan abgetrennt werden. Die Zielwerte im Ethylenprodukt liegen typischwerweise bei weniger als 2 vol.ppm und ggf. sogar bei weniger als 1 vol.ppm. Für denn Fall teilweiser Entfernung im Rahmen der Sauerstoffentfernung (also dann, wenn später noch eine weitere Acetylenhydrierung erfolgt) sind höhere Werte zulässig. In jedem Fall erfolgt aber eine Reduzierung des Acetylengehalts gegenüber dem ursprünglichen Spaltgas.

Für den Fall einer späteren Acetylenhydrierung im Rahmen einer entsprechenden Hydrierung kann ein der Sauerstoffentfernung entnommenes Stoffgemisch einen Gehalt von weniger als 250 vol.ppm, weniger als 100 vol.ppm oder weniger als 10 vol.ppm Acetylen aufweisen. Erfolgt diese spätere Acetylenentfernung nicht, kann ein Acetylengehalt insbesondere weniger als 5 vol.ppm, weniger als 2 vol.ppm, weniger als 1 vol.ppm, weniger als 0,5 vol.ppm, weniger als 0,3 vol.ppm oder weniger als 0,1 vol.ppm betragen. Die Sauerstoffentfernung wird in beiden Fällen derart vorgenommen, dass eine entsprechende Reduktion des Acetylengehaltes erzielt wird. Restgehalte an Sauerstoff können insbesondere bei weniger als 1000 vol.ppm, weniger als 500 vol.ppm, weniger als 100 vol.ppm, weniger als 10 vol.ppm oder weniger als 1 vol.-ppm liegen.

In einer derartigen Ausgestaltung der Erfindung - also insbesondere gemäß der bereits erwähnten "Variante 3" - ist insbesondere vorgesehen, dass der zweite Produktstrom stromauf der Vereinigung einer Kondensatabtrennung und/oder einer Vorverdichtung unterworfen wird und/oder bei dem stromab der Sauerstoffentfernung und stromauf der Demethanisierung ein oder mehrere Verfahrensschritte durchgeführt werden, der oder die aus einer Kohlendioxidentfernung, einer Trocknung und einer Kohlenwasserstofffraktionierung ausgewählt ist oder sind. Auch hier kann also insbesondere eine, neben einer ersten Kohlendioxidentfernung im ersten Produktstrom, zweite Kohlendioxidentfernung durchgeführt werden, um die vorhandenen Kapazitätsgrenzen zur Aufbereitung des ersten Produktstromes nicht zu überschreiten bzw. zu verletzen.

In einer Ausgestaltung der Erfindung ist vorgesehen, dass in der Demethanisierung eine Fraktion gebildet wird, die überwiegend oder ausschließlich Kohlenwasserstoffe mit zwei Kohlenstoffatomen enthält, und die nach oder vor einer Selektivhydrierung von Acetylen einer Trennung der Kohlenwasserstoffe mit zwei Kohlenstoffatomen voneinander unterworfen wird. Eine Selektivhydrierung stromab eines Steamcrackers ist, wie vorliegend erkannt wurde, oftmals kapazitätslimitierend bei einer Nachrüstung bzw. Kapazitätserweiterung. Ist diese aber, wie in Ausgestaltungen der Erfindung der Fall, im Wesentlichen weitgehend stromab insbesondere der Demethanisierung vorgesehen, wird diese nur in geringerem Umfang zusätzlich belastet.

Gemäß einer Ausgestaltung der Erfindung wird in der Selektivhydrierung ein zumindest Palladium enthaltender Katalysator verwendet. Besonders vorteilhafte und technisch übliche Katalysatoren basieren auf Palladium, können aber auch noch mit anderen Elementen dotiert sein, insbesondere Silber, Gold, Cer u.a., um die katalytischen Eigenschaften weiter zu verbessern.

In einer Ausgestaltung der Erfindung ist vorgesehen, dass die oxidative Dehydrierung unter Verwendung eines oder mehrerer, die Metalle Molybdän, Vanadium, Niob und optional Tellur enthaltender Katalysatoren durchgeführt wird. Entsprechende Katalysatorsysteme sind, wie auch nachfolgend noch erläutert, bewährt und robust und erlauben neben einer vorteilhaften Gewinnung von Olefinen auch ggf. die Herstellung von entsprechenden organischen Säuren in Koppelproduktion.

Grundsätzlich kann eine Kohlendioxidentfernung, der beispielsweise in nicht erfindungsgemäßen Vergleichsbeispielen ein Sammelstrom unterworfen wird, in Form einer Laugewäsche durchgeführt werden oder eine solche umfassen. Wie auch nachfolgend erläutert, kann insbesondere dann, wenn vor einer Vereinigung zu einem entsprechenden Sammelstrom - insbesondere gemäß der bereits erwähnten "Variante 1" - eine separate Kohlendioxidentfernung im zweiten Produktstrom vorgesehen sein, so dass die vorhandene Kohlendioxidentfernung nicht übermäßig belastet bzw. zu viel Lauge benötigt wird.

In den nicht erfindungsgemäßen Vergleichsbeispielen kann vorgesehen sein, dass die Kohlendioxidentfernung, der der zweite Produktstrom unterworfen wird - insbesondere gemäß der bereits erwähnten "Variante 1" und gemäß der bereits erwähnten "Variante 2" - oder in den Ausgestaltungen der Erfindung die Kohlendioxidentfernung stromab der Sauerstoffentfernung - insbesondere gemäß der bereits erwähnten "Variante 3" - in Form einer regenerativen Wäsche durchgeführt wird oder eine solche umfasst. Diese regenerative Wäsche kann insbesondere höhere Kohlendioxidgehalte bewältigen und ist hinsichtlich der Betriebsmittel vorteilhaft, reichert aber das entsprechende Gasgemisch typischerweise nicht in dem Umfang an Kohlendioxid ab wie eine Laugewäsche. Ihr kann daher bedarsfweise eine Laugewäsche zur Feinreinigung nachgeschaltet sein.

Gemäß einer Ausgestaltung der Erfindung wird die Sauerstoffentfernung insbesondere derart durchgeführt, dass ein Restsauerstoffgehalt stromab hiervon weniger als 500 vol.-ppm, 250 vol.-ppm, 100 vol.-ppm, 10 vol.-ppm oder 1 vol.-ppm beträgt. Insbesondere ist hier, wie erwähnt, ein höherer Sauerstoffgehalt möglich als typischerweise im Rohgasstrom eines Steamcrackers. Dennoch ist der Wert vorteilhafterweise ausreichend niedrig, um eine sicherheitstechnische Gefährdung durch Anreicherung in einer leichten Fraktion aus Methan, Kohlenmonoxid und anderen leicht siedenden Komponenten zu vermeiden, so dass sich sicherheitstechnische Vorteile ergeben. Ebenso ist sicherzustellen, dass eine ausreichende Abreicherung erfolgt, um ein Fouling, insbesondere bei Anwesenheit von Dienen, zu vermeiden. Weil Sauerstoff nicht extrem tief entfernt werden muss und ein verbleibender Restgehalt in einer Demethanisierung entfernt wird, ergibt sich insbesondere eine Minimierung des Ethylenverlustes in der Rohgasbehandlung.

Bei der Trennung von Kohlenwasserstoffen mit zwei Kohlenstoffatomen wird ein an Ethan angereicherter Strom gewonnen, wobei der an Ethan angereicherte Strom zumindest teilweise zum Steamcracken und/oder zur oxidativen Dehydrierung als Teil des ersten und/oder zweiten Einsatzstroms zurückgeführt wird. Mit einer derartigen Ausgestaltung ist insbesondere eine Kapazitätserweiterung eines Steamcrackers durch Nutzung der oxidativen Dehydrierung insbesondere für einen Ethanrecycle möglich, wie erwähnt.

Gemäß einer Ausgestaltung der Erfindung wird in der Aufbereitung stromab der jeweiligen Vereinigung eine Abkühlung zumindest eines Teils des jeweils bearbeiteten Gasgemischs auf Temperaturen von weniger als -120°C, -135°C oder -150°C vorgenommen. Insbesondere ist eine Nutzung von Spitzenkälte in der Demethanisierung möglich, durch welche sich eine Reduzierung der Ethylenverluste in der leichten Fraktion aus der Demethanisierung ergibt.

Gemäß einer Ausgestaltung der Erfindung wird zumindest ein Teil des zweiten Produktstroms der bereits erwähnten Kondensatabtrennung unterworfen, in der ein Kondensatstrom abgetrennt wird, der mindestens 1 Gew.-% Essigsäure enthält, wobei der Kondensatstrom insbesondere einer weiteren Aufbereitung unterzogen wird, um Essigsäure als Wertprodukt zu gewinnen. Die Erfindung ermöglicht in einer entsprechenden Ausgestaltung eine vorteilhafte Gewinnung dieses Wertprodukts. Wie unten genauer erläutert, kann Essigsäure insbesondere auch zur Erhöhung der Ethylenausbeute hydriert werden

Gemäß einer Ausgestaltung der Erfindung wird dem Steamcracken ein gasförmiger und/oder flüssiger Einsatz zugeführt. Insbesondere bei einem flüssigen Einsatz zum Steamcracken kann der oxidativen Dehydrierung das Ethan zugeführt werden.

Gemäß einer Ausgestaltung der Erfindung kann eine Hydrierung von insbesondere in der oxidativen Dehydrierung gebildeter Essigsäure vorgesehen sein. Alternativ oder zusätzlich kann eine Dehydratisierung von insbesondere in der Dehydrierung der Essigsäure gebildetem Ethanol vorgesehen sein. Hierdurch kann insbesondere die Ethylenausbeute erhöht werden.

Eine Anlage, die zur Durchführung eines Verfahrens in einer beliebigen Ausgestaltung der Erfindung eingerichtet ist, ist ebenfalls Gegenstand der Erfindung und profitiert in gleicher Weise von den vorstehend zu einzelnen Ausgestaltungen erwähnten und nachfolgend noch erläuterten Vorteilen. Es handelt sich hierbei um eine Anlage zur Herstellung eines oder mehrerer Kohlenwasserstoffe, die dafür eingerichtet ist, einen ersten Einsatzstrom unter Erhalt eines ersten Produktstroms einem Steamcracken und einen Ethan enthaltenden zweiten Einsatzstrom unter Erhalt eines zweiten Produktstroms einer oxidativen Dehydrierung zu unterwerfen, unter Verwendung zumindest eines Teils des ersten Produktstroms und unter Verwendung zumindest eines Teils des zweiten Produktstroms einen Demethanisierungseinsatzstrom zu bilden und diesen zumindest zum Teil einer Demethanisierung zu unterwerfen, und bei der Bildung des Demethanisierungseinsatzstromes eine zumindest teilweise Sauerstoffentfernung vorzunehmen, wobei die Anlage dafür eingerichtet ist zumindest ein Teil des ersten Produktstroms unter Erhalt einer leichteren Fraktion und einer schwereren Fraktion separat von dem zweiten Produktstrom einer Deethanisierung oder einer Depropanisierung zu unterwerfen, den Demethanisierungseinsatzstrom unter Vereinigung zumindest eines Teils der leichteren Fraktion und zumindest eines Teils des zweiten Produktstroms zu bilden, und die Sauerstoffentfernung stromab der Vereinigung vorzunehmen. Sie ist ferner dafür eingerichtet, zumindest einen Teil des ersten Produktstroms ohne vorherige Acetylenhydrierung oder unter nur teilweiser Acetylenhydrierung der Vereinigung zuzuführen, und die Sauerstoffentfernung als eine Acetylenentfernung umfassend durchzuführen.

Kurze Beschreibung der Zeichnungen
Figur 1 veranschaulicht ein Verfahren gemäß einem nicht erfindungsgemäßen Vergleichsbeispiel.
Figur 2 veranschaulicht ein Verfahren gemäß einem nicht erfindungsgemäßen Vergleichsbeispiel.
Figur 3 veranschaulicht ein Verfahren gemäß einem nicht erfindungsgemäßen Vergleichsbeispiel.
Figur 4 veranschaulicht ein Verfahren gemäß einem nicht erfindungsgemäßen Vergleichsbeispiel.
Figur 5 veranschaulicht ein Verfahren gemäß einer Ausgestaltung der Erfindung.
Figur 6 veranschaulicht ein Verfahren gemäß einer Ausgestaltung der Erfindung.
Figur 7 veranschaulicht ein Verfahren gemäß einer weiteren Ausgestaltung.

### Ausführliche Beschreibung

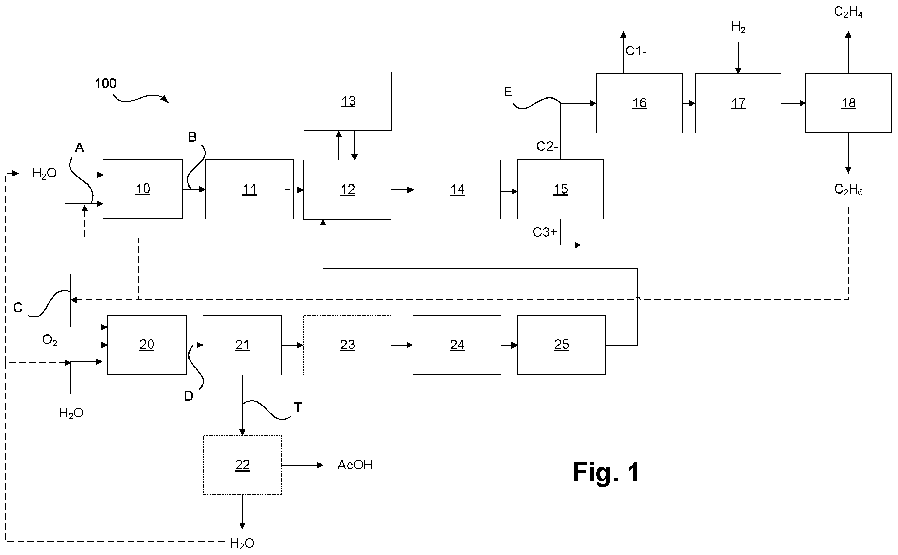

In Figur 1 ist ein Verfahren gemäß einem nicht erfindungsgemäßen Vergleichsbeispiel in Form eines schematischen Ablaufplans veranschaulicht und insgesamt mit 100 bezeichnet.

Als zentrale Verfahrensschritte sind hier ein Steamcracken 10 unter Verwendung eines oder mehrerer Prozesseinheiten wie Spaltöfen und eine oxidative Dehydrierung 20, die unter Verwendung eines oder mehrerer Reaktoren durchgeführt werden kann, veranschaulicht. Wie hier veranschaulicht, wird dem Steamcracken 10 ein hier mit A bezeichneter erster Einsatzstrom zugeführt und dieser dort unter Erhalt eines hier mit B bezeichneten ersten Produktstroms bearbeitet. Dem Steamcracken 10 wird ferner Wasser H₂O in Form von Dampf zugeführt. Ein hier mit C bezeichneter, Ethan enthaltender zweiter Einsatzstrom wird unter Erhalt eines hier mit D bezeichneten zweiten Produktstroms der oxidativen Dehydrierung 20 unterworfen. Der oxidativen Dehydrierung 20 werden ferner Wasser H₂O in Form von Dampf und Sauerstoff O₂ zugeführt. Die Führung der weiteren Stoffströme, die nicht gesondert bezeichnet sind, ergibt sich unmittelbar aus der Darstellung in Form von Flusspfeilen.

Dem Steamcracken 10 ist ein beispielsweise fachüblicher Quenchschritt 11 nachgeschaltet. Stromab des Quenchschritts 11 erfolgt eine Verdichtung 12, eine (üblicherweise) auf einer Zwischenstufe der Verdichtung 12 vorgenommene Entfernung 13 von Kohlendioxid, eine Trocknung 14, eine Deethanisierung 15 unter Gewinnung einer schwereren Fraktion C3+ mit Kohlenwasserstoffen mit drei Kohlenstoffatomen und schwereren Kohlenwasserstoffen und einer leichteren Fraktion C2- mit Kohlenwasserstoffen mit zwei Kohlenstoffatomen, Methan, Kohlenmonoxid und ggf. anderen leicht siedenden Komponenten (wie verbleibendem Sauerstoff), eine Demethanisierung 16 unter Abtrennung einer leichten Fraktion C1- mit Methan und anderen leicht siedenden Komponenten, eine Tail-End-Hydrierung 17, der Wasserstoff H₂ zugeführt wird, und eine Trennung 18 von Kohlenwasserstoffen mit zwei Kohlenstoffatomen voneinander unter Erhalt einer Ethylenfraktion C₂H₄ und einer Ethanfraktion C₂H₆. Letztere kann, wie in Form eines gestrichelten Pfeils veranschaulicht, in das Steamcracken 10 und/oder in die oxidative Dehydrierung 20 zurückgeführt werden.

Der oxidativen Dehydrierung 20 ist eine Abtrennung 21 von Kondensat nachgeschaltet, in der ein Kondensatstrom T gewonnen wird. Dieser wird einer optionalen Kondensataufbereitung 22 zugeführt, in der eine Essigsäurefraktion AcOH und eine Wasserfraktion H₂O gewonnen werden können. Letztere kann, wie in Form eines gestrichelten Pfeils veranschaulicht, in das Steamcracken 10 und/oder in die oxidative Dehydrierung 20 zurückgeführt werden. Der Abtrennung 21 von Kondensat ist eine hier optionale (Vor-)Verdichtung 23 nachgeschaltet, stromab derer sich eine Sauerstoffentfernung 24 und eine Entfernung 25 von Kohlendioxid anschließen. Eine Spurenentfernung 24 kann auch vor einer (Vor-)Verdichtung 23 oder auf einer Zwischenstufe der (Vor-)Verdichtung 23 erfolgen.

Ein stromab der Entfernung 25 von Kohlendioxid vorliegendes Gasgemisch wird in der in Figur 1 veranschaulichten Ausgestaltung 100 in die Verdichtung 12 geführt, die stromauf der Demethanisierung 16 angeordnet ist, so dass ein hier mit E bezeichneter Demethanisierungseinsatzstrom unter Verwendung jeweils eines Teils des ersten Produktstroms B und des zweiten Produktstroms D gebildet wird, und die Sauerstoffentfernung 24 Teil der Aufbereitung des zweiten Produktstroms D ist.

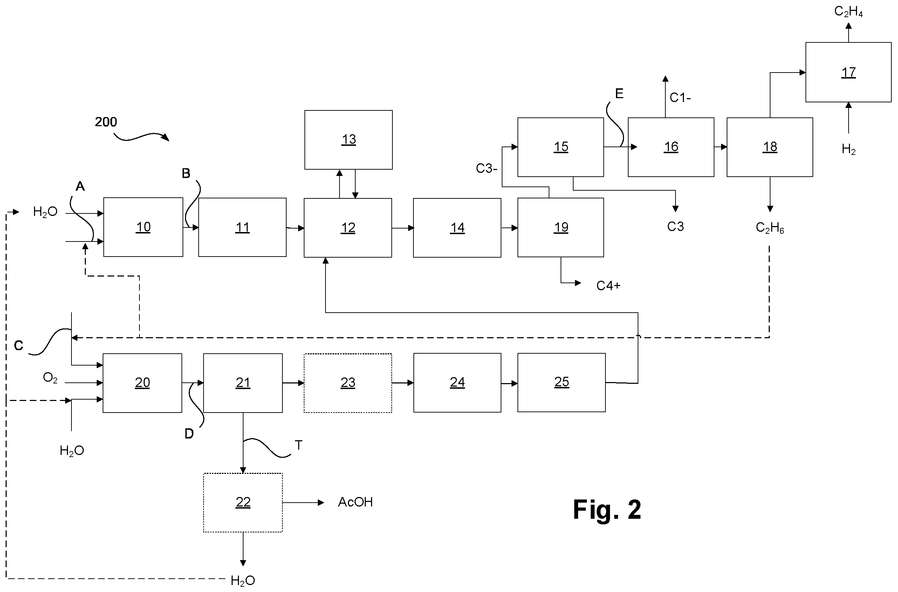

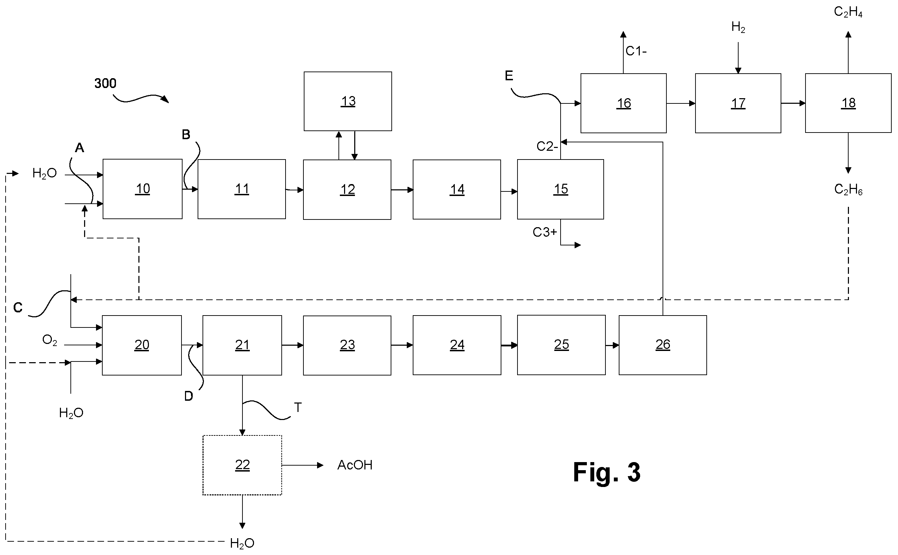

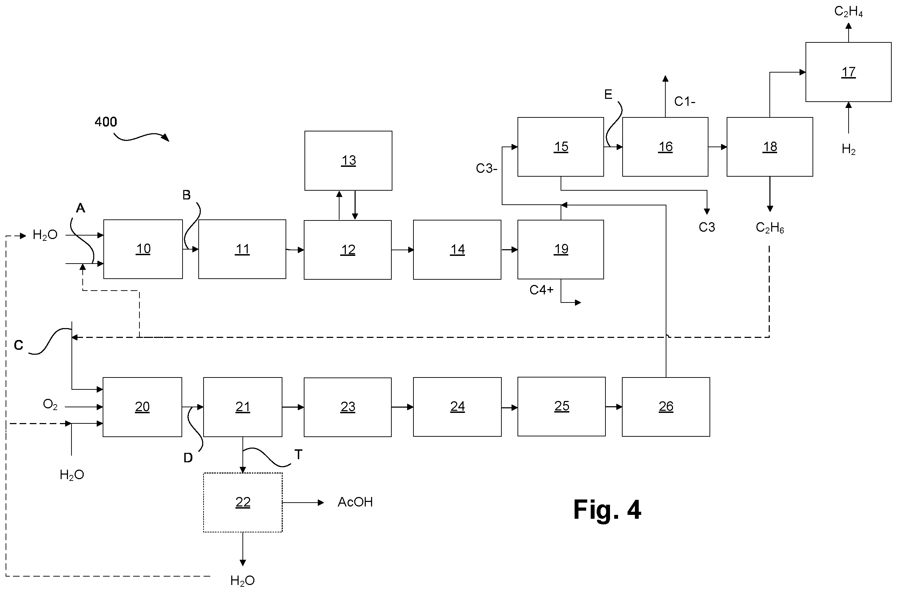

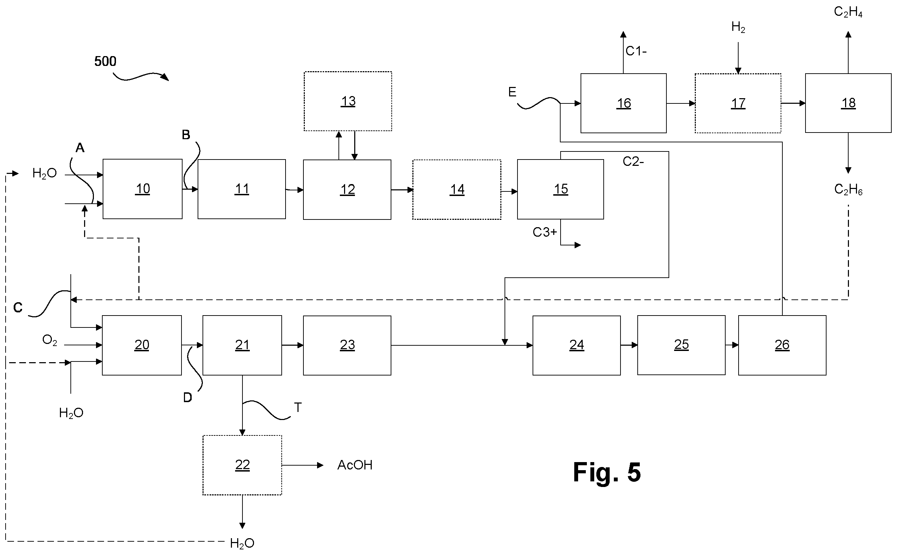

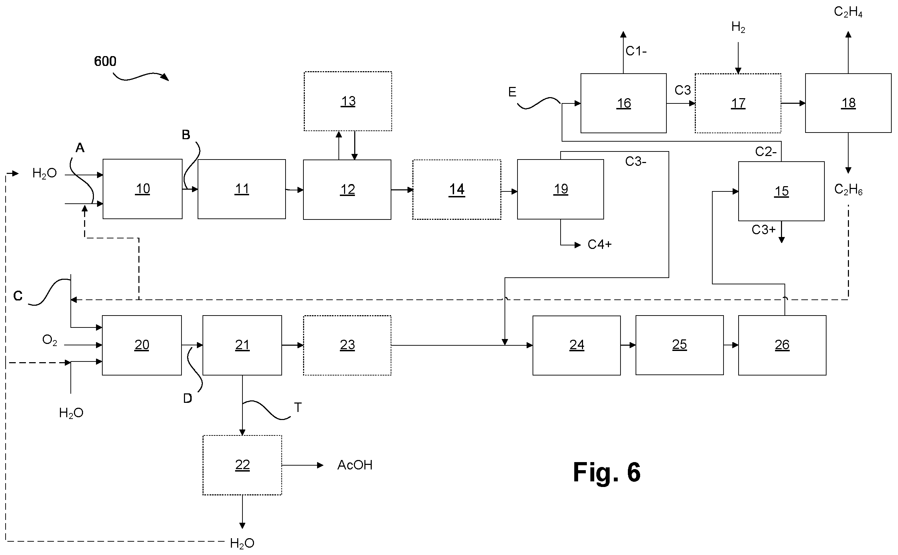

In den Figuren 2 bis 6 sind Verfahren gemäß weiterer nicht erfindungsgemäßer Vergleichsbeispiele (Figuren 2, 3 und 4) sowie Ausgestaltungen der vorliegenden Erfindung (Figuren 5 und 6) veranschaulicht und jeweils insgesamt mit 200 bis 600 bezeichnet. Nachfolgend werden insbesondere die Unterschiede gegenüber der in Figur 1 veranschaulichten Ausgestaltung 100 erläutert. Jeweils optional vorgesehene Komponenten bzw. Verfahrensschritte sind in Form gestrichelter Blöcke dargestellt und werden nachfolgend nicht in allen Fällen als optional erwähnt. Die Ausgestaltungen gemäß den Figuren 1 und 2 entsprechen dabei insbesondere der zuvor mehrfach erwähnten "Variante 1", die Ausgestaltungen gemäß den Figuren 3 und 4 insbesondere der zuvor mehrfach erwähnten "Variante 2" und die Ausgestaltungen gemäß den Figuren 5 und 6 insbesondere der zuvor mehrfach erwähnten "Variante 3".

In der in Figur 2 veranschaulichten Ausgestaltung 200 erfolgt die Zusammenführung grundsätzlich in gleicher Weise wie zu Ausgestaltung 100 gemäß Figur 1 erläutert; ein wesentlicher Unterschied besteht hier in der nachfolgenden Aufbereitung, wie nachfolgend noch genauer erläutert.

In der in Figur 3 veranschaulichten Ausgestaltung 300 ist in der Aufbereitung des zweiten Produktstroms D, d.h. des Produktstroms der oxidativen Dehydrierung 20, eine separate Trocknung 26 vorgesehen, die sich der Entfernung 25 von Kohlendioxid anschließt. Ein hier erhaltenes Gasgemisch wird mit der leichten Fraktion C2- aus der Deethanisierung 16 zusammengeführt. Auch hier wird damit der Demethanisierungseinsatzstrom E unter Verwendung jeweils eines Teils des ersten Produktstroms B und des zweiten Produktstroms D gebildet wird, und die Sauerstoffentfernung 24 ist Teil der Aufbereitung des zweiten Produktstroms D.

Gemäß den Ausgestaltungen 200, 400 und 600, die in den Figuren 2, 4 und 6 veranschaulicht sind, ist stromauf der Deethanisierung 15 jeweils eine Depropanisierung 19 vorgesehen, die unter Gewinnung einer schwereren Fraktion C4+ mit Kohlenwasserstoffen mit vier Kohlenstoffatomen und schwereren Kohlenwasserstoffen und einer leichteren Fraktion C3- mit Kohlenwasserstoffen mit zwei und drei Kohlenstoffatomen, Methan und ggf. anderen leicht siedenden Komponenten durchgeführt wird. Entsprechend enthält die schwere Fraktion C3 aus der sich anschließenden Deethanisierung 15 dann auch nur noch im Wesentlichen Kohlenwasserstoffe mit drei Kohlenstoffatomen aus dem Steamcracken 10.

Gemäß den Ausgestaltungen 200 und 400, die in den Figuren 2 und 4 veranschaulicht sind, ist die Tail-End-Hydrierung 17 stromab der Trennung 18 von Kohlenwasserstoffen mit zwei Kohlenstoffatomen voneinander angeordnet. Sie ist in den in den Figuren 5 und 6 veranschaulichten Ausgestaltungen insbesondere optional und daher in Form gestrichelter Blöcke veranschaulicht.

In der in Figur 4 veranschaulichten Ausgestaltung 400 ist in der Aufbereitung des zweiten Produktstroms D, d.h. des Produktstroms der oxidativen Dehydrierung 20, wie in der Ausgestaltung 300 gemäß Figur 3, eine separate Trocknung 26 vorgesehen, die sich der Entfernung 25 von Kohlendioxid anschließt. Ein hier erhaltenes Gasgemisch wird mit der leichten Fraktion C3- aus der Depropanisierung 19 zusammengeführt. Auch hier wird damit der Demethanisierungseinsatzstrom E unter Verwendung jeweils eines Teils des ersten Produktstroms B und des zweiten Produktstroms D gebildet wird, und die Sauerstoffentfernung 24 ist Teil der Aufbereitung des zweiten Produktstroms D aus der oxidativen Dehydrierung.

In der in Figur 5 veranschaulichten Ausgestaltung 500 wird im Unterschied zu den zuvor erläuterten Ausgestaltungen die leichte Fraktion C2- aus der Deethanisierung, der hier nur Komponenten des ersten Produktstroms B zugeführt werden, der Sauerstoffentfernung 24 unterworfen. Dieser Sauerstoffentfernung 24 werden auch Komponenten des zweiten Produktstroms D, d.h. des Produktstroms der oxidativen Dehydrierung 20, zugeführt. Die Kohlendioxidentfernung 25 und die Trocknung 26 schließen sich an. Ein der Trocknung 26 entnommenes Gasgemisch wird in Form des Demethanisierungseinsatzstroms E der Demethanisierung 16 zugeführt. Auch hier wird damit der Demethanisierungseinsatzstrom E unter Verwendung jeweils eines Teils des ersten Produktstroms B und des zweiten Produktstroms D gebildet wird. Die Sauerstoffentfernung 24 ist Teil der Aufbereitung des ersten Produktstroms B und des zweiten Produktstroms D bzw. vereinigter Teile hiervon.

In der in Figur 6 veranschaulichten Ausgestaltung 600 wird im Unterschied zu den zuvor erläuterten Ausgestaltungen die leichte Fraktion C3- aus der Depropanisierung, der hier nur Komponenten des ersten Produktstroms B zugeführt werden, der Sauerstoffentfernung 24 unterworfen. Dieser Saustoffentfernung 24 werden auch Komponenten des zweiten Produktstroms D, d.h. des Produktstroms der oxidativen Dehydrierung 20, zugeführt. Die Kohlendioxidentfernung 25 und die Trocknung 26 schließen sich an. Ein der Trocknung 26 entnommenes Gasgemisch wird in Form des Demethanisierungseinsatzstroms E der Demethanisierung 16 zugeführt. Auch hier wird damit der Demethanisierungseinsatzstrom E unter Verwendung jeweils eines Teils des ersten Produktstroms B und des zweiten Produktstroms D gebildet wird. Die Sauerstoffentfernung 24 ist Teil der Aufbereitung des ersten Produktstroms B und des zweiten Produktstroms D bzw. vereinigter Teile hiervon.

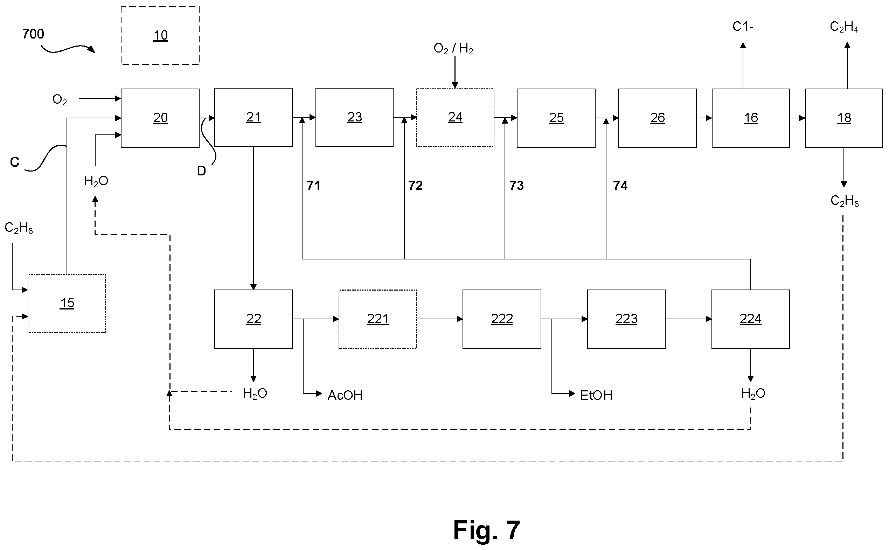

Im Rahmen der Integration mit einem Steamcracker, wo Ethylen ein Hauptprodukt darstellt, kann zudem eine Maximierung der Ethylenausbeute gewünscht sein. Hierbei gilt es insbesondere, Essigsäure als Koppelprodukt nach Möglichkeit zu vermeiden. Hierzu eröffnet eine geeignete Umsetzung von Essigsäure zu Ethylen eine Möglichkeit, die im Folgenden anhand von Figur 7 beispielhaft beschrieben wird. Lediglich aus Gründen der Allgemeingültigkeit der Darstellung wird dabei auf eine Veranschaulichtung des Steamcrackens 10 verzichtet bzw. dieses nur als unverbundener Block 10 gezeigt.

In Figur 7 ist ein Verfahren gemäß einer entsprechenden Ausgestaltung veranschaulicht und insgesamt mit 700 bezeichnet. Die in Figur 7 veranschaulichten Verfahrensschritte sind mit identischen Bezugszeichen wie oben angegeben. Auf eine wiederholte Erläuterung wird an dieser Stelle verzichtet.

Eine Einbindung in ein Kombinationsverfahren kann dabei in jeder beliebigen Weise erfolgen, insbesondere wie zuvor erläutert. Ausgestaltungen der vorliegenden Erfindung betreffen insbesondere entsprechende Kombinationsverfahren. Die hierbei eingesetzten Verfahrensschritte können aber nicht nur in Ausgestaltungen der vorliegenden Erfindung, sondern in allen Kombinationsverfahren zum Einsatz kommen, in denen eine oxidative Dehydrierung 20 mit beliebigen anderen Verfahren, insbesondere, aber nicht beschränkt auf, das Steamcracken 10, kombiniert ist, und in denen entsprechende Produktströme, Teile, Fraktionen und dergleichen in beliebiger Weise oder an beliebigen Positionen zusammengeführt und damit insbesondere bestimmte Verfahrensschritte bzw. Komponenten gemeinsam genutzt werden. Insbesondere kann auch dann, wenn in einer Aufarbeitung eines Produktstroms des Steamcrackens 10 eine Demethanisierung zum Einsatz kommt, in einem Kopfstrom der Demethanisierung enthaltener Wasserstoff, insbesondere nach einer geeigneten Abtrennung, in der Hydrierung genutzt werden.

Wie in Figur 7 veranschaulicht, wird die oxidative Dehydrierung 20, insbesondere von Ethan hier mit einer nachgeschalteten Kondensatabtrennung 21 und einer Kondensataufbereitung 22 betrieben. Es schließen sich im Ethylen- bzw. Olefinweg insbesondere die (Vor-)Verdichtung 23, in Ausgestaltungen der Erfindung eine Sauerstoffentfernung 24, die in beliebiger Weise ausgestaltet sein kann, eine Kohlendioxidentfernung 25 und eine Trocknung 26 an. Eine Demethanisierung 16 ist auch hier vorgesehen. Diese kann insbesondere in der oben veranschaulichten Weise mit einem Steamcracken 10 gekoppelt sein, d.h. an einer geeigneten Stelle stromauf hiervon kann ein Produktstrom B aus einem Steamcracken 10 bzw. Fraktionen, Teile usw. eingespeist werden. Eine Trennung von Kohlenwasserstoffen mit zwei Kohlenstoffatomen ist auch hier mit 18 bezeichnet. Ethan aus dieser Trennung kann insbesondere in eine optionale Deethanisierung 15 geführt werden, wo ein Ethanstrom gebildet wird, der jeweils zuminfest anteilig als der (erste) Einsatzstrom A dem Steamcracken, insbesondere aber als der (zweite) Einsatzstrom C der oxidativen Dehydrierung 20 zugeführt werden kann.

Ein in der Kondensataufbereitung gebildeter Wasserstrom H₂O kann insbesondere in die oxidative Dehydrierung 20 zurückgeführt werden, wie jeder andere stromab hiervon gebildeter Wasserstrom. Essigsäure AcOH kann ausgeschleust werden, wird aber in der hier veranschaulichten Ausgestaltung zumindest zu einem Teil einer optionalen Verdichtung 221 zugeführt und danach in eine Essigsäurehydrierung 222 eingespeist, in der Ethanol EtOH gebildet wird. Das Ethanol kann ausgeschleust werden, wird aber in der hier veranschaulichten Ausgestaltung zumindest zu einem Teil einer Ethanoldehydratisierung 223 zugeführt, in der aus Ethanol Wasser und Ethylen gebildet werden. Nach einer Phasentrennung kann ein Ethylenstrom je nach Bedarf an den Positionen 71 bis 74 in den Ethylenweg geführt werden, wobei die Positionen 71 und 72 erfindungsgemäße Ausgestaltungen darstellen.

Nachfolgend werden nochmals Vorteile und Ausgestaltungen der vorliegenden Erfindung unter expliziter Bezugnahme auf den Stand der Technik erläutert.

Die oxidative Dehydrierung von Alkanen, insbesondere von Ethan, kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die oxidative Dehydrierung kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine in-situ-Regenerierung ermöglicht bzw. bewirkt. Schließlich werden im Gegensatz zum Steamcracken geringere Mengen an wertlosen Nebenprodukten wie Koks gebildet. In Ausgestaltungen der vorliegenden Erfindung ist die oxidative Dehydrierung, insbesondere von Methan, insbesondere deshalb vorteilhaft, weil sie die vorteilhafte Nutzung von Ethan ermöglicht, auch wenn das Steamcracken zur Nutzung (rein oder überwiegend) flüssiger Einsätze ausgelegt ist.

Zu weiteren Details bezüglich der oxidativen Dehydrierung sei auf Fachliteratur, beispielsweise Ivars, F. und Löpez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 12, 2013, Seiten 3196 bis 3247, sowie X. Li, E. Iglesia, Kinetics and Mechanism of Ethane Oxidation to Acetic Acid on Catalysts Based on Mo-V-Nb Oxides, J. Phys. Chem. C, 2008, 125, 15001-15008, verwiesen.

In Ausgestaltungen der vorliegenden Erfindung besonders relevante Aspekte der oxidativen Dehydrierung und weiterer technischer Hintergrund von entsprechenden Ausgestaltungen werden nachfolgend weiter erläutert.

Das Produktgas der oxidativen Dehydrierung von Ethan nach einer Kondensatabtrennung enthält signifikante Mengen an Acetylen, beispielsweise bis zu 500 vol.-ppm, 400 vol.-ppm oder 300 vol.-ppm und mehr als 10 vol.-ppm, 50 vol.-ppm, oder 100 vol.-ppm, an Kohlenmonoxid, beispielsweise bis zu 5 vol%, 4 vol%, oder 3 vol% und mehr als 0,5 vol%, oder 1 vol%, an Kohlendioxid, beispielsweise bis zu 4 vol%, 3 vol% oder 2vol% und mehr als 0,5 vol% oder 1 vol% und an Sauerstoff, beispielsweise bis zu 2 mol%, 1,5 mol%, 1 mol%, oder 0,5 mol% und mehr als 500 vol.-ppm, 1000 vol.-ppm oder 2500 vol.-ppm. Außer Acetylen sind weitere einfach und mehrfach ungesättigten Kohlenwasserstoffe (höhere Olefine, Diene und höhere Acetylene) nur im Spurenbereich im Produktgas enthalten.

Optionen zur Entfernung von Sauerstoff, zuvor auch als "Sauerstoffentfernung" bezeichnet, sind prinzipiell bekannt und beispielsweise in der WO 2020/187572 A1 oder auch in der WO 2018/153831 A1 offenbart. Bei einer entsprechenden Sauerstoffentfernung kann, wie erwähnt, insbesondere auch Acetylen entfernt werden, wie auch unten noch ausgeführt. Dabei können typischerweise Restgehalte an Sauerstoff von weniger als 1000 vol.-ppm, 500 vol.-ppm, 100 vol.-ppm, 10 vol.-ppm oder 1 vol.-ppm, an Acetylen von weniger als 5 vol.-ppm, 2 vol.-ppm, 0,5 vol.-ppm, 0,3 vol.-ppm oder 0,1 vol.-ppm, erreicht werden. In Ausgestaltungen der vorliegenden Erfindung ist eine vollständige Entfernung von Sauerstoff und Acetylen nicht notwendig.

Wie beispielsweise in der WO 2018/153831 A1 beschrieben, können Sauerstoff, Kohlenmonoxid und optional Acetylen aus dem Produktstrom einer oxidativen Dehydrierung von Ethan entfernt werden. Hierbei ist allgemein die Verwendung eines Oxidationskatalysators offenbart, der als Bestandteile insbesondere die Metalle Niob, Kupfer, Zink, Palladium, Silber, Platin, Gold, Eisen, Mangan, Cer, Zinn, Rubidium und Chrom aufweisen kann. Dabei werden bevorzugt kupfer- und/oder platinbasierte Systeme, insbesondere aber kupferbasierte Systeme, verwendet. Ausgestaltungen der vorliegenden Erfindung können unter Verwendung sämtlicher aus dem Stand der Technik und der Fachliteratur bekannter Acetylen- und Sauerstoffentfernungsverfahren durchgeführt werden.

Hinsichtlich der Entfernung von Sauerstoff (hier relevant ohne Berücksichtigung weiterer Komponenten) sind zudem auch weitere Ansätze bekannt. Prinzipiell kann diese bereits unmittelbar am Reaktoraustritt der oxidativen Dehydrierung von Ethan erfolgen, wie z.B. in der WO 2010/115108 A1 bzw. der US 8,519,210 B2 sowie der WO 2019/175731 A1 bzw. der WO 2019/175732 A1 offenbart. Auch eine Entfernung nach einer Essigsäureabscheidung und vor (z.B. gemäß der WO 2018/153831 A1) oder nach (z.B. gemäß der WO 2018/153831 A1 oder der WO 2020/187572 A1) einer Verdichtung ist möglich. Eine entsprechende Entfernung unmittelbar am Reaktoraustritt fällt unter den oben erläuterten Begriff der "Rohgasbehandlung".

Bekannt ist auch die Einspeisung von Wasserstoff in eine solche Rohgasbehandlung des Produktgases einer oxidativen Dehydrierung von Ethan. Gemäß Stand der Technik wird immer nur der Prozessgasstrom einer oxidativen Dehydrierung von Ethan einer solchen Rohgasbehandlung unterworfen (im Gegensatz insbesondere zu der mehrfach erwähnten "Variante 3"). In der Rohgasbehandlung können dabei insbesondere Kupferoxid enthaltende Katalysatoren oder zumindest eines der Elemente Kupfer, Silber, Gold, Mangan, Zink, Nickel, Platin, Palladium, Rhodium, Iridium und/oder Ruthenium enthaltende Katalysatoren verwendet werden.

Zumeist erfolgt, wie erwähnt, bei einer entsprechenden Sauerstoffentfernung zugleich eine Reduzierung/Entfernung von Acetylen und auch Kohlenmonoxid. In Ausgestaltungen der vorliegenden Erfindung tritt die Entfernung von Acetylen jedoch in den Hintergrund (für "Variante 1" und "Variante 2", bedingt für "Variante 3"). Der Umsatz von Kohlenmonoxid und die Bildung von Kohlendioxid sind jedoch relevant für Prozesskonzepte gemäß entsprechenden Ausgestaltungen der Erfindung (resultierende nachgeschaltete Kohlendioxid-Entfernung). Insbesondere für Acetylen kann jedoch üblicherweise eine spezifikationsgerechte Entfernung von Acetylen erreicht werden (wichtig für Variante 3 ohne weitere C2-Hydrierung).

Bei der oxidativen Dehydrierung, insbesondere bei Einsatz der genannten Katalysatorsysteme, entsteht Essigsäure als Koppelprodukt (typisches Verhältnis 3 bis 12 mol/mol Ethylen zu Essigsäure), der Bedarf an Essigsäure ist jedoch oftmals limitiert auf spezielle stromabwärtige Produkte bzw. Verfahren (z.B. die Herstellung von Vinylacetatmomomer, VAM). Essigsäure kann als Bestandteil der stromab der oxidativen Dehydrierung abgetrennten Kondensatphase (der typische Essigsäuregehalt im Kondensat liegt bei 1 bis 30 Gew.-%, 3 bis 25 Gew.-% oder 5 bis 20 Gew.-%) abgetrennt werden und durch eine geeignete Aufbereitung als eigenes Wertprodukt bereitgestellt werden, wie erläutert.

Andererseits entstehen im Gegensatz zum Steamcracker bei der oxidativen Dehydrierung von Ethan - abgesehen von den wesentlichen Nebenprodukten Kohlenmonoxid und Kohlendioxid - aber eben auch nur Ethylen und Essigsäure als Produkte. Somit entfällt auch die Anforderung einer geeigneten Verwertung weiterer Produktfraktionen aus der oxidativen Dehydrierung und der entsprechenden apparativen Einrichtungen im Zerlegungsteil. In Ausgestaltungen der vorliegenden Erfindung ist eine Nutzung entsprechender Produkte besonders vorteilhaft möglich.

Die beim Steamcracken eingesetzte Technologie ist ebenfalls umfangreich vorgeschrieben. Wichtig ist hier typischerweise insbesondere die strikte Einhaltung der Produktspezifikation von Ethylen in Hinblick auf stromabwärtige Prozesse (z.B. Polyethylenherstellung, Ethylenoxidherstellung etc.). Hier sind neben anderen insbesondere für Kohlenmonoxid, Kohlendioxid, Sauerstoff und Acetylen sehr strenge Grenzwerte einzuhalten, die durch entsprechende Reinigungen und Trennschritte erreicht werden. Besonders relevante Prozessschritte sind nachfolgend aufgeführt und näher erläutert.

In Ausgestaltungen der vorliegenden Erfindung sind insbesondere bestimmte Komponenten des Produktgemischs bzw. Rohgasstroms beim Steamcracken und Aspekte von dessen weiterer Behandlung besonders relevant. Insbesondere umfasst ein entsprechendes Produktgemisch einen hohen Anteil an mehrfach ungesättigten Kohlenwasserstoffen (Diene und Acetylene) und weist einen geringen Kohlenmonoxid- und Kohlendioxidgehalt auf. Insbesondere liegt der Gehalt an Kohlenmonoxid im Rohgasstrom, d.h. im Wesentlichen unmittelbar stromab eines entsprechenden Reaktors bzw. dem sich anschließenden Quench, bei weniger als 0,50 vol.-%, 0,20 vol.-% oder 0,10 vol.-%. Typische Gehalte von Kohlendioxid im Rohgasstrom (vor einer Laugewäsche) liegen insbesondere bei weniger als 0,05 vol.-%, weniger als 0,02 vol.-% oder weniger als 0,01 vol.-%. Der Sauerstoffgehalt soll ausgesprochen gering sein bzw. soll im Wesentlichen kein Sauerstoff enthalten sein. Der Sauerstoffgehalt liegt insbesondere bei weniger als 10 vol.-ppm, insbesondere bei weniger als 1 vol.-ppm im Ethylenprodukt aufgrund von strikten Produktspezifikation von Ethylen in Hinblick auf stromabwärtige Prozesse (z.B. Herstellung von Polyethylen, Ethylenoxid etc.). Aber auch im Rohgasstrom des Steamcrackers ist es wichtig, den Sauerstoffgehalt zu limitieren. Dies resultiert insbesondere aus der Wechselwirkung mit mehrfach ungesättigten Kohlenwasserstoffen (Fouling, insbesondere bei Verdichtung und auch im Sumpf von Kolonnen).

Prinzipiell kommen unterschiedliche Einsatzstoffe für das Steamcracken in Betracht. Diese umfassen leichte Einsätze, wie Ethan, Propan und sogenanntes Liquefied Petroleum Gas (LPG), insbesondere in sogenannten Gascrackern, aber auch schwerere Einsätze wie Naphtha oder sogenanntes Atmospheric Gasoil (AGO) etc., insbesondere in sogenannten Flüssigcrackern. Entsprechend dem Einsatzstoff sind die Cracker optimiert und unterscheiden sich z.T. in ihrem Design (z.B. Rohgashydrierung für Gascracker und Frontendhydrierung für Flüssigcracker). Beim Steamcracken wird insbesondere ein breites Produktspektrum (Ethylen, Propylen, insbesondere in Flüssigcrackern jedoch auch Aromaten, höhere Kohlenwasserstoffe und schwerere Fraktionen) erhalten. Ein Recycle von höheren Kohlenwasserstoffen, insbesondere von Paraffinen, in die Spaltöfen ist möglich. In Ausgestaltungen der Erfindung ist insbesondere der Ethanrecycle relevant. Aspekte der Behandlung eines Rohgases aus dem Steamcracken umfassen die Entfernung von Kohlenmonoxid mittels Demethanisierung (siehe unten) und/oder die Entfernung von Kohlendioxid, üblicherweise mittels Laugewäsche (siehe unten).

Eine Selektivhydrierung von Kohlenwasserstoffen mit zwei Kohlenstoffatomen dient dazu, den Acetylengehalt spezifikationsgerecht zu reduzieren. Dabei wird Acetylen über geeigneten Katalysatoren selektiv zu Ethylen hydriert. Die Selektivhydrierung erfolgte früher überwiegend mit nickelbasierten Katalysatoren, heutzutage werden vornehmlich palladiumbasierte Katalysatoren mit geeigneten Dotierungen wie Silber, Gold, Cer u.a. eingesetzt. Es besteht eine beträchtliche Sensitivität gegenüber Kohlenmonoxid. Kohlenmonoxid dient als Moderator und der Kohlenmonoxidgehalt in der Hydrierung liegt typischerweise im Bereich von 50 bis 2500 vol.-ppm, 100 bis 1500 vol.-ppm oder 150 bis 1000 vol.-ppm. Diese Kohlenmonoxidgehalte liegen deutlich unterhalb der o.g. Kohlenmonoxidgehalte eines Prozessgases einer oxidativen Dehydrierung von Ethan und auch geringer als in Fällen, in denen eine Rohgasbehandlung eingesetzt wird.

Hohe Kohlenmonoxidgehalte in der Selektivhydrierung können dabei bedingt durch eine höhere Temperatur ausgeglichen werden, dies führt jedoch ggf. zu Ethylenverlust. Die maximale Temperatur ist dabei einerseits durch die Designtemperatur des Reaktors, insbesondere bei bestehenden Anlagen, aber auch durch das Betriebsfenster des Katalysators begrenzt. Wird der o.g. Temperaturbereich überschritten, lässt sich die Selektivhydrierung nicht mehr betreiben bzw. es ist der Einsatz besonders angepasster Katalysatoren erforderlich.

Grundsätzlich ist die Selektivhydrierung auch nicht unempfindlich gegenüber der Anwesenheit von Sauerstoff, da bei Anwesenheit von Sauerstoff Wasser gebildet werden kann, was wiederum die Bildung von weiteren Nebenprodukten wie Carbonsäuren und Grünöl fördert. In Ausgestaltungen der vorliegenden Erfindung werden entsprechende Probleme in besonders vorteilhafter Weise ausgeräumt.

Als Positionen für eine Selektivhydrierung sind verschiedene Varianten bekannt:
(1) Eine Rohgashydrierung ist unmittelbar nach Verdichtung, Laugewäsche und Trocknung positioniert. Diese kommt vorzugsweise in Gascrackern, also mit gasförmigen Einsätzen betriebenen Steamcrackern zur Anwendung. Dabei werden ebenfalls höhere mehrfach ungesättigte Kohlenwasserstoffe zumindest anteilig umgesetzt (insbesondere Butadien, Methylacetylen und Propadien). Typische Kohlenmonoxidgehalte liegen im unteren Bereich der o.g. Spanne, also insbesondere im Bereich von 50 bis 1000 vol.-ppm oder 50 bis 500 vol.-ppm.
(2) Eine sogenannte Frontendhydrierung ist nach einer Deethanisierung positioniert. Hier handelt es sich also um eine reine Acetylenhydrierung. In beiden Fällen sind neben Ethan zudem entsprechende Mengen an Methan, Wasserstoff und Kohlenmonoxid im Feedstrom der Selektivhydrierung enthalten. Typische Kohlenmonoxidgehalte liegen hier verfahrensbedingt höher als bei der Rohgashydrierung, also insbesondere im Bereich von 100 bis 2500 vol.-ppm oder 100 bis 1500 vol.-ppm. Als Variante ist auch die Anordnung nach einer Depropanisierung bekannt, in diesem Fall umfasst der Feedstrom der Hydrierung auch Kohlenwasserstoffe mit drei Kohlenstoffatomen. Ähnlich wie bei der Rohgashydrierung werden dann die entsprechenden höheren mehrfach ungesättigten Kohlenwasserstoffe zumindest anteilig umgesetzt.
(3) Prinzipiell ist auch eine so genannte Tailendhydrierung bekannt, bei der vor der Hydrierung zumindest noch der ein Strom mit Methan und anderen leichten Komponenten abgetrennt wird und entsprechend eine stöchiometrische Wasserstoffdosierung erforderlich ist. Diese Variante ist in Ausgestaltungen der vorliegenden Erfindung besonders relevant, bei der vor einer entsprechenden Tailendhydrierung zumindest noch eine leichte Fraktion C1- in der Demethanisierung abgetrennt wird und entsprechend eine stöchiometrische Wasserstoffzudosierung erforderlich ist. Zusammen mit dieser leichten Fraktion werden also leichtere Moleküle und somit insbesondere auch Kohlenmonoxid und Sauerstoff aus dem Prozessstrom entfernt, so dass diese keine Auswirkung auf die Tailendhydrierung haben. Auch bei einer Tailendhydrierung kommen Edelmetallkatalysatoren zum Einsatz, die bereits erwähnt wurden und insbesondere palladiumbasiert sein können.

Neben der Anforderung an die hohe Produktreinheit des Ethylenproduktes machen auch kryogene Anlagenteile eine quantitative Abscheidung von Kohlendioxid notwendig, um ein Ausfrieren von Kohlendioxid und damit Verlegungen zu vermeiden.

Kohlendioxid kann - insbesondere in Gehalten, wie sie bei der oxidativen Dehydrierung von Ethan auftreten - aufgrund seiner hohen Wechselwirkung mit geeigneten Lösungsmitteln bzw. Waschflüssigkeiten ebenso vergleichsweise einfach aus dem Produktgemisch entfernt werden, wobei bekannte Verfahren zur Kohlendioxidentfernung, insbesondere entsprechende Wäschen (beispielsweise Aminwäschen) zum Einsatz kommen können. Die beladene Waschflüssigkeit wird dann in einer separaten Kolonne regeneriert und dabei wird sehr reines Kohlendioxid durch Desorption freigesetzt.

Sollten Folgeschritte die Abwesenheit oder nur eine sehr geringe Restkonzentration von Kohlendioxid erfordern (z.B. aufgrund einer Katalysatorinhibierung oder sogenannten Katalysatorvergiftung), kann der Restgehalt an Kohlendioxid nach einer Aminwäsche bedarfsweise durch eine optionale Laugewäsche als Feinreinigung anforderungsgemäß weiter reduziert werden.

Mit gewissen Ausnahmen können die entsprechenden Waschflüssigkeiten auch mit Sauerstoff reagieren, wodurch es im Laufe der Zeit zu einer nachteiligen Alterung bzw. Schädigung der Waschmittel kommen kann, die einen kontinuierlichen Purge- und Makeup-Strom erfordern bzw. zu einer unerwünschten Verkürzung der Lebensdauer dieser Waschflüssigkeiten führen. Daher ist auch aus diesem Aspekt die Entfernung von Sauerstoff stromauf einer entsprechenden Wäsche vorteilhaft.

Im Rohgasstrom eines Steamcrackers liegen typischerweise deutlich geringere Kohlendioxidgehalte als bei der oxidativen Dehydrierung vor. Diese werden üblicherweise mit einer Laugewäsche entfernt. Ein um mehrere Größenordnungen höherer Kohlendioxidgehalt wie bei der oxidativen Dehydrierung von Ethan würde wie zuvor ausgeführt zu einem übermäßigen Laugeverbrauch führen. In Ausgestaltungen der vorliegenden Erfindung wird daher wie erwähnt insbesondere eine separate Abscheidung von Kohlendioxid mittels Amin- und optional Laugewäsche stromab der oxidativen Dehydrierung vorgenommen.

Die Entfernung von Wasser erfolgt gemäß Stand der Technik mittels regenerativer Trockner auf Molsiebbasis und ist neben der Erreichung der Produktspezifikation ebenfalls zwingend in Hinblick auf nachfolgende kryogene Prozesschritte, um dort Verlegungen durch Abscheidung von Eis und Hydraten zu vermeiden.

Typischerweise muss im Rahmen einer geeigneten Verfahrensführung sowohl bei einer oxidativen Dehydrierung von Ethan, als auch im Zerlegungsteil eines Steamcrackers auch im Produktstrom enthaltenes Methan (z.B. insbesondere aus dem Ethaneinsatzstrom der oxidativen Dehydrierung stammend) entfernt werden. Dazu kommt üblicherweise eine Demethanisierung zum Einsatz, die entsprechende kryogene Bedingungen erfordert. Eine Demethanisierung entfernt dabei gleichzeitig Kohlenmonoxid und Wasserstoff aus dem entsprechenden Strom. Es wird also eine mit C1- (sprich "C1minus") bezeichnete Fraktion gebildet, die als wesentliche Bestandteile Methan, Wasserstoff und/oder Kohlenmonoxid enthält. Im Eintrittsstrom der Demethanisierung noch enthaltene Spuren von Sauerstoff gelangen dabei ebenfalls in diese Fraktion. Daher wird üblicherweise angestrebt, den Eintrittsgehalt an Sauerstoff im Eintrittsstrom zur Demethanisierung zu begrenzen und so die mögliche Bildung einer explosionsfähigen Atmosphäre am Kopf zu vermeiden. Eine entsprechende Auslegung des Demethanizers ist zwar möglich, wie beispielsweise in der WO 2018/082945 A1 beschrieben, bedeutet aber entsprechend deutlich erhöhten apparativen Aufwand. Die EP 3 456 703 A1 beschreibt eine Demethanisierung im Zerlegungsteil einer Anlage zur oxidativen Dehydrierung von Ethan, die mit einer Druckwechseladsorption im Kopfstrom kombiniert wird. Zur Minimierung von Produktverlusten wird des Weiteren angestrebt, den Ethylengehalt im Kopfgas des Demethanizers zu minimieren.

Schlussendlich muss nicht umgesetztes Ethan von Ethylen getrennt werden, was mittels eines mehrfach erwähnten C2-Splitters, der ebenfalls unter kryogenen Bedingungen betrieben wird, erfolgt. Dieser muss also so gebaut und betrieben werden, dass Ethan im Ethylen praktisch quantitativ entfernt wird (geforderter Schlussendlich muss nicht umgesetztes Ethan von Ethylen getrennt werden, was mittels eines C2-Splitters, der ebenfalls unter kryogenen Bedingungen betrieben wird, erfolgt. Dieser muss also so gebaut und betrieben werden, dass Ethan im Ethylen praktisch quantitativ entfernt wird (geforderter Reinheitsgrad des Ethylen-Produkts i.d.R. mehr als 99,9 %) und gleichzeitig ein Ethanstrom, der zur oxidativen Dehydrierung zurückgeführt wird, möglichst wenig bzw. kein Ethylen enthält.

Ansätze zur Integration von Steamcracker und oxidativer Dehydrierung von Ethan sind grundsätzlich bekannt, wie bereits erwähnt. Allen eingangs genannten Schriften ist gemeinsam, dass sie nicht auf die wohlbekannte Entstehung von Essigsäure als Koppelprodukt der oxidativen Dehydrierung eingehen und jeweils nur eine wässrige Kondensatphase abtrennen. Ausgestaltungen der vorliegenden Erfindung berücksichtigen dagegen insbesondere auch diesen Aspekt.

Die WO 2018/024650 A1 fokussiert insbesondere auf die Integration eines Steamcrackers mit Ethaneinsatz mit einer oxidativen Dehydrierung von Ethan. Die Einspeisung eines Prozessgases aus einer oxidativen Dehydrierung von Ethan in den Zerlegungsteil eines Steamcrackers wird grundsätzlich beschrieben und beansprucht. Es sind jedoch keine Lösungsansätze offenbart, welche die Problematik der deutlich abweichenden Sauerstoff-, Kohlenmonoxid- und Acetylengehalte im Prozessgas aus einer oxidativen Dehydrierung von Ethan und in entsprechenden Strömen im Zerlegungsteil eines Steamcrackers aufgreifen und lösen. Eine Tailendhydrierung, und eine Zusammenführung von Produktströmen stromauf dieser Tailendhydrierung ist beschrieben, so dass die Kohlenwasserstoffe mit zwei Kohlenstoffatomen aus Steamcracker und oxidativen Dehydrierung von Ethan also einer gemeinsamen Hydrierung unterzogen werden. Dies steht genau im Gegensatz zum Lösungsansatz von Ausgestaltungen der vorliegenden Erfindung. Dementsprechend ist auch keine Rohgasbehandlung des Prozessstromes einer oxidativen Dehydrierung von Ethan dargestellt oder beansprucht.

Die WO 2014/134703 A1 offenbart eine Sauerstoffentfernung unmittelbar stromab eines Reaktors zur oxidativen Dehydrierung von Ethan (sogenannter "Afterburner"), es werden typische Restsauerstoffgehalte von weniger als 1000 vol.-ppm genannt. Zwar werden in allgemeiner Weise Integrationsansätze von oxidativer Dehydrierung von Ethan und Steamcracken beansprucht, die wahlweise die Prozesseinheiten C2-Splitter und/oder eine Acetylenhydrierung umfassen. Dabei kann die Integration sowohl explizit stromauf als auch explizit stromab der Acetylenhydrierung erfolgen. Jedoch werden auch hier keine Lösungen für die Problematik der deutlich abweichenden Kohlenmonoxid- und Acetylengehalte im Prozessgas aus einer oxidativen Dehydrierung von Ethan und in entsprechenden Strömen im Zerlegungsteil eines Steamcrackers aufgezeigt. Auch die vorteilhafte Positionierung eines Demethanizers und Integration im Gesamtverfahren wird in der Schrift nicht näher betrachtet.

Die CN 103086821 B betrifft die Integration eines Naphthacrackers mit einer oxidativen Dehydrierung von Ethan. Es finden sich zwar grundsätzliche Ausführungen zur Entfernung von Sauerstoff, Kohlenmonoxid und Kohlendioxid. Jedoch offenbart die Schrift keine Lösung für die Entfernung von Acetylen. Vielmehr lehrt diese Schrift im Gegensatz zu den zuvor gemachten Ausführungen, dass bei der oxidativen Dehydrierung von Ethan von Ethan gerade keine Nebenprodukte wie Essigsäure und Acetylen entstehen sollen.

Ausgestaltungen der vorliegenden Erfindung erfüllen zuvor dargestellten gegensätzlichen Anforderungen und ermöglichen eine optimierte Integration einer oxidativen Dehydrierung von Ethan und eines Steamcrackers.

Eine Reihe von Aspekten steht nämlich der Einspeisung eines Produktgases aus der oxidativen Dehydrierung von Ethan in den Zerlegungsteil eines Steamcrackers entgegen. So ist ein hoher Kohlenmonoxidgehalt im Prozessgas der oxidativen Dehydrierung von Ethan limitierend für die Hydrierung von Kohlenwasserstoffen mit zwei Kohlenstoffatomen; hier kann zunächst nur eine so genannte Tailendhydrierung zur Anwendung kommen bzw. es können nur spezielle Katalysatoren verwendet werden, die hohe Kohlenmonoxidgehalt tolerieren. Ein zu hoher Kohlendioxidgehalt im Prozessgas der Kohlenmonoxidgehalt spricht gegen eine Entfernung mittels typischer Laugewäsche. Der Sauerstoffgehalt im Prozessgas der oxidativen Dehydrierung kann zu Foulingeffekten, einer möglichen Anreicherung von Sauerstoff in der erwähnten C1 minus-Fraktion, und daher zu einer sicherheitstechnischen Gefährdung führen. Dabei bleibt der Sauerstoffgehalt auch nach einer gesonderten Rohgasbehandlung im Prozessgas der oxidativen Dehydrierung ggf. trotzdem kritisch wegen mehrfach ungesättigter Kohlenwasserstoffe aus dem Cracker bzw. es stellt sich hier eine sehr scharfe Reinheitsanforderung (weniger als 10 vol.-ppm, insbesondere weniger als 1 vol.-ppm Sauerstoff) an eine solche Rohgasbehandlung, die entsprechenden Aufwand bedingt und zu Ethylenverlusten führen kann.

Ausgestaltungen der Erfindung lösen diese Probleme in der erläuterten Weise.

Wird Sauerstoff in den zuvor erläuterten nicht erfindungsgemäßen Vergleichsbeispielen bereits im Prozessgas der oxidativen Dehydrierung von Ethan mittels einer Rohgasbehandlung weitestgehend entfernt, ist eine Einspeisung stromauf einer Laugewäsche möglich ("Variante 1"). Diese Laugewäsche kann dann insbesondere auch zur Entfernung von Kohlendioxidresten im Prozessgas aus der oxidativen Dehydrierung von Ethan (Feinreinigung) dienen. Wird nur eine teilweise Reduzierung des Sauerstoffgehalts im Prozessgas der oxidativen Dehydrierung von Ethan erreicht, kann die Einspeisung insbesondere stromab einer Deethanisierung bzw. Depropanisierung ("Variante 2") erfolgen, um Foulingreaktionen unter Beteiligung von Sauerstoff und Dienen zu vermeiden.

In Ausgestaltungen der Erfindung ist vorgesehen, dass die Sauerstoffentfernung erst nach der erwähnten Vereinigung erfolgt ("Variante 3"). Hierbei ist insbesondere die vorgeschlagene Vereinigung stromab mindestens einer Deethanisierung bzw. Depropanisierung des Produktstroms des Steamcrackens gefolgt von einer Rohgasbehandlung des kombinierten Prozessstroms besonders vorteilhaft.

Insbesondere bei "Variante 2" und "Variante 3" kann verbleibender Sauerstoff über einen folgenden Demethanizer entfernt werden. Verunreinigungen durch Acetylen können jeweils zumindest anteilig über die Rohgasbehandlung reduziert oder entfernt werden, eine finale spezifikationsgerechte Entfernung ist mittels einer erwähnten Selektivhydrierung möglich, die gemäß Ausgestaltungen der Erfindung insbesondere als eine Tailendhydrierung ausgeführt sein kann (d.h. nach Abtrennung von Kohlenmonoxid in der Demethanisierung).

Nachfolgend werden die gemäß nicht erfindungsgemäßen Vergleichsbeispielen und Ausgestaltungen der vorliegenden Erfindung insbesondere gemäß "Variante 1", "Variante 2" und "Variante 3" vorgesehenen Ausgestaltungen nochmals zusammengefasst.

Eine Vorbehandlung des zweiten Produktstroms der oxidativen Dehydrierung von Ethan (nur "Variante 1" und "Variante 2", die "Variante 3" gemäß Ausgestaltungen der Erfindung erfordert vorteilhafterweise keine gesonderte Vorbehandlung des zweiten Produktstroms) umfasst insbesondere eine Sauerstoffreduzierung durch eine Rohgasbehandlung (dabei erfolgt zugleich eine Reduzierung von Acetylen und Kohlenmonoxid) sowie insbesondere eine Vorverdichtung (für "Variante 1" optional) stromauf und/oder stromab der Rohgasbehandlung. Ferner ist in einer entsprechenden Vorbehandlung des zweiten Produktstroms der oxidativen Dehydrierung von Ethan insbesondere eine separate Kohlendioxidentfernung vorgesehen (insbesondere mittels Aminwäsche; hier wird vorteilhafterweise Kohlendioxid als eigenes reines Produkt erhalten zur möglichen weiteren Verwendung und/oder Carbon Capture & Storage). Bei "Variante 1" erfolgt insbesondere nur eine Kohlendioxidentfernung mittels Aminwäsche und dann eine gemeinsame Feinreinigung nach Vereinigung mit dem ersten Produktstrom aus dem Steamcracker. Für "Variante 2" kann die Kohlendioxidentfernung aus dem des zweiten Produktstrom der oxidativen Dehydrierung von Ethan insbesondere auch zweistufig ausgeführt werden (mit regenerativer bzw. Aminwäsche und Laugewäsche), um vor Vereinigung mit dem ersten Produktstrom aus dem Steamcracker ähnlich niedrige Kohlendioxidgehalte wie in letzterem stromab der Laugewäsche zu erreichen und wie für die Einspeisung in kryogene Anlagenteile bzw. für die Einhaltung der Ethylen-Produktspezifikation erforderlich (i.d.R. Werte von weniger als 1 mol-ppm oder noch geringer). Ferner ist in einer entsprechenden Vorbehandlung des zweiten Produktstroms der oxidativen Dehydrierung von Ethan (nur in "Variante 2") insbesondere eine Trocknung vorgesehen.

Wie erwähnt, erfolgt eine Vereinigung der entsprechenden Produktströme bzw. von Teilen hiervon gemäß nicht erfindungsgemäßen Vergleichsbeispielen stromauf einer Demethanisierung, und zwar gemäß "Variante 1" stromauf einer Kohlendioxidentfernung (insbesondere Laugewäsche, hier bevorzugt genutzt als gemeinsame Feinreinigung), wobei, wie mehrfach erwähnt, vorab insbesondere eine entsprechende Vorreinigung des zweiten Produktgemischs der oxidativen Dehydrierung von Ethan erfolgt. Die Vereinigung erfolgt insbesondere optional stromauf einer Verdichtung oder einzelner Verdichterstufen, die einer entsprechenden Kohlendioxidentfernung vorgeschaltet ist bzw. sind, und es schließt sich an die gemeinsame Kohlendioxidentfernung insbesondere eine Trocknung an.

Gemäß der nicht erfindungsgemäßen "Variante 2" und der "Variante 3" gemäß Ausgestaltungen der vorliegenden Erfindung erfolgt eine entsprechende Vereinigung insbesondere stromab einer Deethanisierung oder Depropanisierung des ersten Produktgemischs aus dem Steamcracken, da hier keine Diene (insbesondere Butadien) mehr vorliegen. Je nach Ausführung ist hier eine Trocknung des gemeinsamen Stromes stromab der zuvor genannten Trennung möglich (insbesondere im Falle der Einspeisung stromab einer Depropanisierung).

Zusätzlich ist (insbesondere nur für "Variante 3" gemäß Ausgestaltungen der Erfindung) eine Behandlung des gemischten Stromes nach der Vereinigung mittels einer Rohgasbehandlung im oben erläuterten Sinne vorgesehen, die mindestens den Sauerstoffgehalt reduziert. Insbesondere werden dabei auch Acetylen und Kohlenmonoxid zumindest teilweise umgesetzt und Acetylen vorteilhafterweise bereits spezifikationsgerecht entfernt. Ferner ist hier insbesondere eine Kohlendioxidentfernung vorgesehen, um Verlegungen aufgrund von Ausfrieren von Kohlendioxid im kryogenen Teil zu vermeiden und den Maximalgehalt von Kohlendioxid in der Ethylen-Produktspezifikation einzuhalten.

Notwendige Anpassungen der Temperaturniveaus sind hier und in den vorstehend erläuterten Figuren 1 und 2 nicht explizit berücksichtig und erfolgen auf dem Fachmann bekannte Art und Weise (z.B. Wärmetauscher).

In einer Ausgestaltung der Erfindung werden besonders bevorzugt zumindest Teile des vereinigten Produktgasstroms an einzelnen Stellen des Prozesses bis auf Temperaturen von weniger als -120°C, -135°C oder -150°C gekühlt. Das Erreichen besonders niedriger Temperaturen begünstigt die Minimierung von Ethylenverlusten in der leichten Fraktion C1-, die wie erwähnt im Demethanizer aus dem vereinigten Produktgasstrom abgetrennt wird.

Hieraus ergibt sich ein zusätzlicher Synergievorteil der Integration, da die bei einer eigenständigen, nicht integrierten Anlage zur oxidativen Dehydrierung von Ethan anteilsmäßig nur eine kleine C1 minus-Fraktion zur Verfügung steht. Diese ist nicht tauglich für die Erzeugung nutzbarer Spitzenkälte und es können in einer eigenständigen Anlage zur oxidativen Dehydrierung Temperaturen unterhalb von - 110°C nicht ohne erheblichen zusätzlichen Aufwand erreicht werden. Diese Situation verbessert sich signifikant, wenn man den Produktgasstrom der oxidativen Dehydrierung von Ethan mit dem des Crackers vereinigt und einem gemeinsamen Demethanizer zuführt. So können zumindest anteilig für die Fraktion der oxidativen Dehydrierung von Ethan durch die Verfügbarkeit von Spitzenkälte im o.g. Temperaturbereich Ethylenverluste reduziert werden.

Eine Erhöhung der Ethylenausbeute ist durch Konversion von Essigsäure aus einem stromab der oxdiativen Dehydrierung abgeschiedenen Kondensat zu Ethylen möglich. Entsprechende Verfahrensschritte können, wie erwähnt, nicht nur in Ausgestaltungen der vorliegenden Erfindung, sondern in allen Kombinationsverfahren zum Einsatz kommen, in denen die oxidative Dehydrierung mit beliebigen anderen Verfahren, insbesondere, aber nicht beschränkt auf, das Steamcracken, kombiniert ist, und in denen entsprechende Produktströme, Teile, Fraktionen und dergleichen in beliebiger Weise oder an beliebigen Positionen zusammengeführt und damit insbesondere bestimmte Verfahrensschritte bzw. Komponenten gemeinsam genutzt werden.

Bei der oxidativen Dehydrierung werden, wie erwähnt, insbesondere bei Verwendung von MoVNbOₓ-basierten und insbesondere MoVNbTeOₓ-Katalysatoren unter industriell relevanten Reaktionsbedingungen signifikante Mengen der jeweiligen Carbonsäuren der eingesetzten Paraffine als Nebenprodukte gebildet. Für einen wirtschaftlichen Anlagenbetrieb ist eine entsprechende Koppelproduktion von Olefinen und der jeweiligen Carbonsäuren bei Verwendung des beschriebenen Katalysatortyps in der Regel erforderlich. Dies gilt insbesondere für die Herstellung von Ethylen durch die oxidative Dehydrierung von Ethan, bei der gleichzeitig Essigsäure gebildet wird.

Im Rahmen entsprechender Ausgestaltung ist insbesondere die erwähnte Bildung von Essigsäure als Koppelprodukt relevant. Auf die obigen Ausführungen wird verwiesen. Eine Einstellung des Verhältnisses von Ethylen zu Essigsäure aus der oxidativen Dehydrierung von Ethan ist insbesondere durch eine Anpassung des Wasseranteils, insbesondere des Wasserpartialdrucks im Prozessgasstrom, aber nur innerhalb gewisser Grenzen möglich (vgl. z.B. WO 2018/115416 A1 bzw. EP 3 519 377 B1). Ferner ist ein gewisser Mindestwasseranteil zur Gewährleistung einer stabilen Katalysatorperformance vorteilhaft bzw. erforderlich (vgl. z.B. WO 2018/115418 A1 bzw. EP 3 558 910 B1). Der Einsatz einer oxidativen Dehydrierung von Ethan ist aufgrund der Koppelproduktion zumeist begrenzt auf kleine bis mittlere Anlagenkapazitäten und erfordert eine entsprechende Verwertung der produzierten Essigsäure. Andererseits entstehen wie bereits erwähnt im Gegensatz zum Steamcracken - abgesehen von den wesentlichen Nebenprodukten Kohlenmonoxid und Kohlendioxid - aber eben auch nur Ethylen und Essigsäure als Produkte. Somit entfällt auch die Anforderung einer geeigneten Verwertung weiterer Produktfraktionen aus der oxidativen Dehydrierung von Ethan und der entsprechenden apparativen Einrichtungen im Zerlegungsteil.

Eine Anlage zur oxidativen Dehydrierung von Ethan umfasst typischerweise die bereits mehrfach erläuterten Prozessschritte. Hierbei kann die in der oxidativen Dehydrierung von Ethan gebildete Essigsäure zunächst zu Ethanol hydriert und danach das Ethanol zu Ethylen dehydratisiert werden. Das gebildete Ethylen kann an beliebigen Positionen stromab der oxidativen Dehydrierung von Ethan in ein entsprechendes Verfahren bzw. eine entsprechende Aufbereitungssequenz eingespeist werden.

Die Hydrierung von Carbonsäuren ist bekannt und wird z.B. im Artikel "Hydrogenation and Dehydrogenation" in Ullmann's Encyclopedia of Industrial Chemistry, Ausgabe 2012, beschrieben. Als Coprodukt der Hydrierung wird Wasser gebildet. In dem genannten Artikel wird insbesondere zur Erfordernis von erhöhtem Druck und erhöhter Temperatur für die Hydrierung von Carbonsäuren ausgeführt. Insbesondere Rhenium, Ruthenium, Kupfer und Chrom werden als Katalysatorbestandteile aufgeführt. Entsprechende konkrete Ausführungsformen der Hydrierung finden sich des Weiteren z.B. in EP 0 100 406 B1 unter Verwendung von cobalthaltigen Katalysatoren bei 10 bis 350 bar und 210 bis 330 °C in der Gasphase. Als weitere mögliche Katalysatorbestandteile werden u.a. Mangan und Molybdän aufgeführt.

Die WO 2010/014153 A2 basiert ebenfalls auf einem Cobalt-Katalysator, dabei finden jedoch weitere Bestandteile Verwendung, insbesondere ausgewählt aus Palladium, Platin, Rhodium, Rubidium, Rhenium, Iridium, Cer, Kupfer, Zinn, Molybdän, Wolfram, Vanadium und Zink. Die WO 2011/056597 A2 offenbart über die Katalysatorzusammensetzung hinaus auch Details zur Verfahrensführung der Essigsäurehydrierung und weiteren Aufbereitung des Ethanolproduktes. Auch beispielsweise die WO 2011/097190 A3 offenbart Einzelheiten zur Verfahrensführung mit dem Ziel einer maximierten Ethanolausbeute. Die WO 2013/101373 A1 offenbart einen Ethylacetatrecylce in der Essigsäurehydrierung. Zusätzlich zu den zuvor benannten möglichen Katalysatorbestandteilen werden hier noch Eisen, Lanthan, Cer und Gold benannt. In der WO 2013/122645 A1 kommen überdies auch noch Nickel, Osmium und Cäsium hinzu.

Die Hydrierung von Essigsäure in wässriger Phase wird in Y. Zhao et al., Catalysts 2020, 10, 1270 ff. beschrieben. Es kommt ein Ruthenium-Zinn/Titandioxid-basierter Katalysator zum Einsatz. Insbesondere bei Temperaturen unterhalb von 220°C werden sehr hohe Ethanol-Ausbeuten weit über 90% beschrieben. Die Veröffentlichung enthält weitere Verweise zur Hydrierung von Essigsäure in wässriger Phase.

Die vorgenannten Schriften umfassen also sowohl den Einsatz von reiner oder hochkonzentrierter Essigsäure als Reaktionseinsatz als auch den Einsatz von wässrigen Lösungen von Essigsäure. Die genannten Katalysatoren können dabei sowohl als Vollmaterial oder aber auch als geträgerte Katalysatoren, z.B. auf Materialien, die Dialuminiumtrioxid, Siliciumdioxid, Zirkondioxid, Titandioxid, Kohlenstoff etc. enthalten, zum Einsatz kommen.

Die Dehydratisierung von Alkoholen wie Ethanol an geeigneten Katalysatoren zur Herstellung der entsprechenden Olefine ist ebenfalls bekannt und beispielsweise in der DE 10 2019 119 540 A1 beschrieben. Insbesondere die Herstellung von Ethylen (aus Ethanol) ist gängig und gewinnt im Zusammenhang mit den zunehmenden Produktionsmengen an (Bio-)Ethanol an Bedeutung. Beispielsweise sei hier auch auf den Artikel "Propanols" in Ullmann's Encyclopedia of Industrial Chemistry sowie Intratec Solutions, "Ethylene Production via Ethanol Dehydration", Chemical Engineering 120, 2013, 29 verwiesen. Die Dehydratisierung ist in Gegenwart von Mineralsäurekatalysatoren bei Raumtemperatur oder darüber sehr leicht zu durchzuführen. Die Reaktion selbst ist endotherm und gleichgewichtslimitiert. Hohe Konversionen werden durch niedrige Drücke und hohe Temperaturen begünstigt. Technisch kommen üblicherweise mehrere in Serie geschaltete Adiabatreaktoren mit entsprechenden Zwischenerwärmungen zum Einsatz.

Typischerweise werden dabei heterogene Katalysatoren basierend auf Dialuminiumtrioxid oder Siliciumdioxid verwendet. Allgemein sind mehrere Arten von sauren Katalysatoren geeignet und auch z.B. Molekularsiebe und Zeolithe können verwendet werden. Typische Temperaturen liegen im Bereich von 200 bis 250 °C für die Dehydratisierung von Ethanol oder bei 300 bis 400 °C für die Dehydratisierung von 2-Propanol oder Butanol. Aufgrund der Gleichgewichtslimitierung wird typischerweise der Produktstrom abgetrennt (Abtrennung des Olefinprodukts und auch zumindest teilweise des Wassers durch z.B. Destillation) und der Strom, der unkonvertierten Alkohol enthält, wird zum Reaktoreintritt zurückgeführt. Auf diese Weise können insgesamt sehr hohe Selektivitäten und Ausbeuten erzielt werden. Entsprechende Ausführungsformen insbesondere für höhere Alkohole sind z.B. in der WO 2015/181302 A1 beschrieben. Die Dehydratisierung von Ethanol bei erhöhtem Druck von 25 bis 80 bar ist Gegenstand der EP 2 740 718 A1, während beispielsweise die DE 10 2011 102 971 A1 die vorteilhafte Druckerhöhung des Ethanolfeeds einer Dehydratiseirung mittels einer Pumpe offenbart.

Sämtliche der erläuterten Techniken können zur Lösung der Aufgabe, nicht erwünschte Essigsäure als Koppelprodukt der oxidativen Dehydrierung zielgerichtet zu verwerten. Darüber hinaus lässt sich die Menge an Essigsäure praktisch beliebig reduzieren, so dass nur der aktuell tatsächliche Bedarf an Essigsäure z.B. in einem integrierten Anlagenkomplex bereitgestellt wird.

Damit entfällt insbesondere die Limitierung durch die Koppelproduktion, da das Verhältnis Ethylen zu Essigsäure nur innerhalb gewisser Grenzen durch Variation der Prozessbedingungen, insbesondere des Wasseranteiles im Reaktionseinsatz oder auch der Verweilzeit und des Druckes, einstellbar ist. Konventionelle Verfahren zur Ethylenerzeugung (insbesondere Steamcracking mit befeuerten Öfen) sind mit entsprechenden Kohlendioxidemissionen verbunden.

Durch eine geschickte Kombination der Verfahrensschritte oxidative Dehydrierung von Ethan, optional Aufbereitung von Essigsäure, Essigsäurehydrierung und optional Ethanoldehydratisierung wird eine bedarfsgerechte und praktisch frei wählbare Reduzierung der Essigsäuremenge erreicht. Dabei kann einerseits Ethanol als wertvolles zusätzliches Produkt gewonnen werden oder aber das Ethanol weiter zu Ethylen dehydratisiert werden.

In einer Ausgestaltung kann für die Hydrierung zumindest anteilig Wasserstoff aus einer Elektrolyse eingesetzt werden. Dies ermöglicht die Nutzung von Strom aus regenerierbaren Quellen und Vermeidung zusätzlicher Kohlendioxidemissionen. Entsprechend der Erfordernis kann die Essigsäure vor der Hydrierung aufkonzentriert werden, grundsätzlich ist aber auch die Hydrierung in wässriger Phase bekannt und möglich. Eine dabei ggf. notwendige Druckerhöhung insbesondere für die Hydrierung kann aufgrund des niedrigen apparativen und energetischen Aufwandes dabei besonders vorteilhaft durch eine Pumpe erreicht werden.

Auch die Dehydratisierung kann besonders vorteilhaft bei erhöhtem Druck erfolgen, so dass stromab der Dehydratisierung keine weitere Druckerhöhung des Ethylenstroms aus der Dehydratisierung notwendig ist.

Ausgestaltungen sind bereits oben zu Figur 7 erläutert. Dabei sind unterschiedliche Varianten 71 bis 74 zur Einspeisung einer in der Dehydratisierung gewonnenen Ethylenfraktion aufgezeigt, nämlich stromauf einer Verdichtung oder einzelner Verdichterstufen, stromauf einer Rohgasbehandlung (optional), stromauf einer Kohlendioxidentfernung und/oder stromauf einer Trocknung.

Die dargestellte Rohgasbehandlung ist optional und kann entfallen oder z.B. durch eine Selektivhydrierung an geeigneter Stelle im Prozess ersetzt oder ergänzt werden. Die Rohgasbehandlung kann auch an anderer Stelle, insbesondere vor der Verdichtung oder vor einzelnen Verdichterstufen erfolgen. Bei einer Einspeisung an den Positionen 71, 72 und 73 wird jeweils eine Kohlendioxidentfernung durchlaufen. Gemäß Variante 73 ist eine Druckerhöhung nach der Dehydratisierung nicht erforderlich. Die Positionen von Demethanizer und Splitter können grundsätzlich auch vertauscht sein, wie bereits zuvor ausgeführt.

Eine bei den Varianten 72, 73 und 74 optional erforderliche separate Druckerhöhung des Ethylenstromes nach der Dehydratisierung und Phasentrennung ist nicht dargestellt. Es kann jedoch auf eine solche Verdichtung dieses Stromes verzichtet werden und es kann eine Druckerhöhung im Einsatz der Essigsäurehydrierung und/oder der Ethanoldehydratisierung zum Einsatz kommen. Dort kann die Druckerhöhung vorteilhafterweise insbesondere in einer Flüssigphase mittels einer Pumpe erfolgen.

In einer weiteren Ausgestaltung der Erfindung kommt eine dem Reaktor zur oxidativen Dehydrierung vorgeschaltete Deethanisierung zum Einsatz, um schwerere Komponenten aus dem Reaktionseinsatz der oxidativen Dehydrierung abzutrennen. Diese kann vorteilhafterweise dazu genutzt werden, auch höhere Komponenten aus dem Produktstrom der Dehydratisierung abzutrennen, da sich diese im Sumpfstrom eines C2-Splitters anreichern.

## Patentansprüche

1. Verfahren (400-600) zur Herstellung eines oder mehrerer Kohlenwasserstoffe, bei dem ein erster Einsatzstrom (A) unter Erhalt eines ersten Produktstroms (B) einem Steamcracken (10) und ein Ethan enthaltender zweiter Einsatzstrom (C) unter Erhalt eines zweiten Produktstroms (D) einer oxidativen Dehydrierung (20) unterworfen werden, wobei unter Verwendung zumindest eines Teils des ersten Produktstroms (B) und unter Verwendung zumindest eines Teils des zweiten Produktstroms (D) ein Demethanisierungseinsatzstrom (E) gebildet wird, der zumindest zum Teil einer Demethanisierung (16) unterworfen wird, und bei dem bei der Bildung des Demethanisierungseinsatzstromes (E) eine zumindest teilweise Sauerstoffentfernung (24) vorgenommen wird, wobei zumindest ein Teil des ersten Produktstroms (B) unter Erhalt einer leichteren Fraktion (C2-, C3-) und einer schwereren Fraktion (C3+, C4+) separat von dem zweiten Produktstrom (D) einer Deethanisierung (15) oder einer Depropanisierung (19) unterworfen wird, wobei der Demethanisierungseinsatzstrom (E) unter Vereinigung zumindest eines Teils der leichteren Fraktion (C2-, C3-) und zumindest eines Teils des zweiten Produktstroms (D) gebildet wird, wobei die Sauerstoffentfernung (24) stromab der Vereinigung vorgenommen wird, und wobei zumindest ein Teil des ersten Produktstroms (B) ohne vorherige Acetylenhydrierung oder unter nur teilweiser Acetylenhydrierung der Vereinigung zugeführt wird, und bei dem die Sauerstoffentfernung (24) eine Acetylenentfernung umfasst.

2. Verfahren (100, 200) nach Anspruch 1, bei dem unter Vereinigung zumindest eines Teils des ersten Produktstroms (B) und zumindest eines Teils des zweiten Produktstroms (D) ohne vorherige Abtrennung gasförmiger Kohlenwasserstoffe ein Sammelstrom gebildet und zumindest zum Teil einer Kohlendioxidentfernung (13) unterworfen wird, wobei der Demethanisierungseinsatzstrom (E) unter Verwendung zumindest eines Teils eines der Kohlendioxidentfernung (13) entnommenen Entnahmestroms gebildet wird.

3. Verfahren (400, 600) nach Anspruch 1 oder 2, bei dem der zweite Produktstrom (D) stromauf der Vereinigung einer Kondensatabtrennung (21) und/oder einer Vorverdichtung (23) unterworfen wird und/oder bei dem stromab der Sauerstoffentfernung (24) und stromauf der Demethanisierung (16) ein oder mehrere Verfahrensschritte (25, 26, 15) durchgeführt werden, der oder die aus einer Kohlendioxidentfernung (25), einer Trocknung (26) und einer Kohlenwasserstofffraktionierung (15) ausgewählt ist oder sind.

4. Verfahren (400-600) nach einem der vorstehenden Ansprüche, bei dem in der Demethanisierung (16) eine Fraktion gebildet wird, die überwiegend oder ausschließlich Kohlenwasserstoffe mit zwei Kohlenstoffatomen enthält, und die nach oder vor einer Selektivhydrierung (17) von Acetylen einer Trennung (18) der Kohlenwasserstoffe mit zwei Kohlenstoffatomen voneinander unterworfen wird.

5. Verfahren (400-600) nach einem der vorstehenden Ansprüche, bei dem die oxidative Dehydrierung (20) unter Verwendung eines oder mehrerer, die Metalle Molybdän, Vanadium, Niob und optional Tellur enthaltender Katalysatoren durchgeführt wird.

6. Verfahren (400-600) nach einem der vorstehenden Ansprüche, bei dem die Kohlendioxidentfernung (25) stromab der Sauerstoffentfernung (24) in Form einer regenerativen Wäsche durchgeführt wird oder eine solche umfasst.

7. Verfahren (400-600) nach einem der vorstehenden Ansprüche, bei dem die Sauerstoffentfernung (24) unter Verwendung eines oder mehrerer Katalysatoren durchgeführt wird, der oder die ein oder mehrere Elemente aufweist oder aufweisen, das oder die aus Kupfer, Silber, Gold, Mangan, Zink, Platin, Palladium, Rhodium, Iridium und Ruthenium ausgewählt ist oder sind.

8. Verfahren (400-600) nach einem der vorstehenden Ansprüche, bei dem die Sauerstoffentfernung (24) derart durchgeführt wird, dass sich in einer in der Demethanisierung gebildeten leichten Fraktion kein explosives Gemisch ergibt.

9. Verfahren (400-600) nach einem der vorstehenden Ansprüche, das eine Hydrierung (222) von insbesondere in der oxidativen Dehydrierung (20) gebildeter Essigsäure und/oder eine Dehydratisierung (223) von insbesondere in der Dehydrierung (222) der Essigsäure gebildetem Ethanol umfasst.

10. Anlage zur Herstellung eines oder mehrerer Kohlenwasserstoffe, die dafür eingerichtet ist, einen ersten Einsatzstrom (A) unter Erhalt eines ersten Produktstroms (B) einem Steamcracken (10) und einen Ethan enthaltenden zweiten Einsatzstrom (C) unter Erhalt eines zweiten Produktstroms (D) einer oxidativen Dehydrierung (20) zu unterwerfen, unter Verwendung zumindest eines Teils des ersten Produktstroms (B) und unter Verwendung zumindest eines Teils des zweiten Produktstroms (D) einen Demethanisierungseinsatzstrom (E) zu bilden und diesen zumindest zum Teil einer Demethanisierung (16) zu unterwerfen, und bei der Bildung des Demethanisierungseinsatzstromes (E) eine zumindest teilweise Sauerstoffentfernung (24) vorzunehmen, wobei die Anlage dafür eingerichtet ist zumindest ein Teil des ersten Produktstroms (B) unter Erhalt einer leichteren Fraktion (C2-, C3-) und einer schwereren Fraktion (C3+, C4+) separat von dem zweiten Produktstrom (D) einer Deethanisierung (15) oder einer Depropanisierung (19) zu unterwerfen, den Demethanisierungseinsatzstrom (E) unter Vereinigung zumindest eines Teils der leichteren Fraktion (C2-, C3-) und zumindest eines Teils des zweiten Produktstroms (D) zu bilden, und die Sauerstoffentfernung (24) stromab der Vereinigung vorzunehmen, wobei die Anlage dafür eingerichtet ist, zumindest einen Teil des ersten Produktstroms (B) ohne vorherige Acetylenhydrierung oder unter nur teilweiser Acetylenhydrierung der Vereinigung zuzuführen, und die Sauerstoffentfernung (24) als eine Acetylenentfernung umfassend durchzuführen.

11. Anlage nach Anspruch 10, die zur Durchführung eines Verfahrens nach einem der Ansprüche 1 bis 9 eingerichtet ist.

## Claims

1. Method (400-600) for producing one or more hydrocarbons, wherein a first feed stream (A) is subjected to steam cracking (10) to obtain a first product stream (B) and a second feed stream (C) containing ethane is subjected to oxidative dehydrogenation (20) to obtain a second product stream (D), using at least a portion of the first product stream (B) and using at least a portion of the second product stream (D), a demethanization feed stream (E) being formed, which is subjected to demethanization (16) at least in part, and wherein at least partial oxygen removal (24) is carried out during the formation of the demethanization feed stream (E), at least a portion of the first product stream (B) being subjected to deethanization (15) or depropanization (19) separately from the second product stream (D) to obtain a lighter fraction (C2-, C3-) and a heavier fraction (C3+, C4+), the demethanization feed stream (E) being formed by combining at least a portion of the lighter fraction (C2-, C3-) and at least a portion of the second product stream (D), the oxygen removal (24) being carried out downstream of the combining step, and at least a portion of the first product stream (B) being fed to the combining step without prior acetylene hydrogenation or with only partial acetylene hydrogenation, and wherein the oxygen removal (24) comprises an acetylene removal.

2. Method (100, 200) according to claim 1, wherein a collective stream is formed by combining at least a portion of the first product stream (B) and at least a portion of the second product stream (D) without prior separation of gaseous hydrocarbons, and is subjected at least in part to carbon dioxide removal (13), the demethanization feed stream (E) being formed using at least a portion of a withdrawal stream taken from the carbon dioxide removal (13).

3. Method (400, 600) according to claim 1 or 2, wherein the second product stream (D) is subjected to condensate separation (21) and/or pre-compression (23) upstream of the step of combining, and/or wherein one or more method steps (25, 26, 15) are carried out downstream of the oxygen removal (24) and upstream of the demethanization (16), which step(s) is or are selected from carbon dioxide removal (25), drying (26) and hydrocarbon fractionation (15).

4. Method (400-600) according to any of the preceding claims, wherein a fraction is formed in the demethanization (16) which predominantly or exclusively contains hydrocarbons having two carbon atoms and which is subjected to a separation (18) of the hydrocarbons having two carbon atoms from one another after or before a selective hydrogenation (17) of acetylene.

5. Method (400-600) according to any of the preceding claims, wherein the oxidative dehydrogenation (20) is carried out using one or more catalysts containing the metals molybdenum, vanadium, niobium and optionally tellurium.

6. Method (400-600) according to any of the preceding claims, wherein the carbon dioxide removal (25) downstream of the oxygen removal (24) is carried out in the form of a regenerative scrubbing or comprises such.

7. Method (400-600) according to any of the preceding claims, wherein the oxygen removal (24) is carried out using one or more catalysts comprising one or more elements selected from copper, silver, gold, manganese, zinc, platinum, palladium, rhodium, iridium and ruthenium.

8. Method (400-600) according to any of the preceding claims, wherein the oxygen removal (24) is carried out in such a way that no explosive mixture results in a light fraction formed in the demethanization.

9. Method (400-600) according to any of the preceding claims, which comprises a hydrogenation (222) of acetic acid formed in particular in the oxidative dehydrogenation (20) and/or a dehydration (223) of ethanol formed in particular in the dehydrogenation (222) of the acetic acid.

10. System for producing one or more hydrocarbons, which is designed to subject a first feed stream (A) to steam cracking (10) to obtain a first product stream (B) and a second feed stream (C) containing ethane to oxidative dehydrogenation (20) to obtain a second product stream (D), to form a demethanization feed stream (E) using at least a portion of the first product stream (B) and using at least a portion of the second product stream (D) and to subject this to demethanization (16) at least in part, and to carry out at least a partial removal of oxygen (24) during the formation of the demethanization feed stream (E), wherein the system is designed to subject at least a portion of the first product stream (B) to deethanization (15) or depropanization (19) separately from the second product stream (D) to obtain a lighter fraction (C2-, C3-) and a heavier fraction (C3+, C4+), to form the demethanization feed stream (E) by combining at least a portion of the lighter fraction (C2-, C3-) and at least a portion of the second product stream (D), and to carry out the oxygen removal (24) downstream of the combining step, wherein the system is designed to feed at least a portion of the first product stream (B) to the combining step without prior acetylene hydrogenation or with only partial acetylene hydrogenation, and to carry out the oxygen removal (24) as comprising an acetylene removal.

11. System according to claim 10, which is designed to carry out a method according to any of claims 1 to 9.

## Revendications

1. Procédé (400-600) pour la production d'un ou de plusieurs hydrocarbures, dans lequel un premier flux d'alimentation (A) est soumis à un vapocraquage (10) pour obtenir un premier flux de produits (B) et un second flux d'alimentation (C) contenant de l'éthane est soumis à une déshydrogénation oxydante (20) pour obtenir un second flux de produits (D), dans lequel, en utilisant au moins une partie du premier flux de produits (B) et en utilisant au moins une partie du second flux de produits (D), un flux d'alimentation de déméthanisation (E) est formé, lequel est soumis au moins en partie à une déméthanisation (16), et dans lequel, lors de la formation du flux d'alimentation de déméthanisation (E), une élimination de l'oxygène (24) au moins partielle est effectuée, dans lequel au moins une partie du premier flux de produits (B) est soumise à une déséthanisation (15) ou à une dépropanisation (19) séparément du second flux de produits (D) pour obtenir une fraction plus légère (C2-, C3-) et une fraction plus lourde (C3+, C4+), dans lequel le flux d'alimentation de déméthanisation (E) est obtenu en combinant au moins une partie de la fraction plus légère (C2-, C3-) et au moins une partie du second flux de produits (D), dans lequel l'élimination de l'oxygène (24) est effectuée en aval de la combinaison, et dans lequel au moins une partie du premier flux de produits (B) est amenée à la combinaison sans hydrogénation préalable de l'acétylène ou avec une hydrogénation seulement partielle de l'acétylène, et dans lequel l'élimination de l'oxygène (24) comprend une élimination de l'acétylène.

2. Procédé (100, 200) selon la revendication 1, dans lequel, en combinant au moins une partie du premier flux de produits (B) et au moins une partie du second flux de produits (D) sans séparation préalable d'hydrocarbures gazeux, un flux de collecte est formé et soumis au moins en partie à une élimination du dioxyde de carbone (13), dans lequel le flux d'alimentation de déméthanisation (E) est formé en utilisant au moins une partie d'un flux de prélèvement prélevé à l'élimination du dioxyde de carbone (13).

3. Procédé (400, 600) selon la revendication 1 ou 2, dans lequel le second flux de produits (D) est soumis, en amont de la combinaison, à une séparation des condensats (21) et/ou à une précompression (23) et/ou dans lequel, en aval de l'élimination de l'oxygène (24) et en amont de la déméthanisation (16), une ou plusieurs étapes de procédé (25, 26, 15) sont réalisées, lesquelles sont choisies parmi une élimination du dioxyde de carbone (25), un séchage (26) et un fractionnement des hydrocarbures (15).

4. Procédé (400-600) selon l'une des revendications précédentes, dans lequel une fraction est formée lors de la déméthanisation (16), laquelle fraction contient majoritairement ou exclusivement des hydrocarbures comportant deux atomes de carbone et est soumise à une séparation (18) des hydrocarbures comportant deux atomes de carbone les uns des autres après ou avant une hydrogénation sélective (17) de l'acétylène.

5. Procédé (400-600) selon l'une des revendications précédentes, dans lequel la déshydrogénation oxydante (20) est réalisée à l'aide d'un ou de plusieurs catalyseurs contenant les métaux molybdène, vanadium, niobium et éventuellement tellure.

6. Procédé (400-600) selon l'une des revendications précédentes, dans lequel l'élimination du dioxyde de carbone (25) en aval de l'élimination de l'oxygène (24) est réalisée sous la forme d'un lavage régénératif ou comprend un tel lavage.

7. Procédé (400-600) selon l'une des revendications précédentes, dans lequel l'élimination de l'oxygène (24) est réalisée en utilisant un ou plusieurs catalyseurs qui présentent un ou plusieurs éléments choisis parmi cuivre, argent, or, manganèse, zinc, platine, palladium, rhodium, iridium et ruthénium.

8. Procédé (400-600) selon l'une des revendications précédentes, dans lequel l'élimination de l'oxygène (24) est réalisée de manière à ne pas produire de mélange explosif dans une fraction légère formée lors de la déméthanisation.

9. Procédé (400-600) selon l'une des revendications précédentes, comprenant une hydrogénation (222) de l'acide acétique formé en particulier lors de la déshydrogénation oxydante (20) et/ou une déshydratation (223) de l'éthanol formé en particulier lors de la déshydrogénation (222) de l'acide acétique.

10. Installation pour la production d'un ou de plusieurs hydrocarbures, conçue pour soumettre un premier flux d'alimentation (A) à un vapocraquage (10) pour obtenir un premier flux de produits (B) et un second flux d'alimentation (C) contenant de l'éthane à une déshydrogénation oxydante (20) pour obtenir un second flux de produits (D), former un flux d'alimentation de déméthanisation (E) en utilisant au moins une partie du premier flux de produits (B) et en utilisant au moins une partie du second flux de produits (D) et soumettre ledit flux d'alimentation de déméthanisation au moins en partie à une déméthanisation (16), et effectuer une élimination de l'oxygène (24) au moins partielle lors de la formation du flux d'alimentation de déméthanisation (E), dans laquelle l'installation est conçue pour soumettre, séparément du second flux de produits (D), au moins une partie du premier flux de produits (B) à une déséthanisation (15) ou à une dépropanisation (19) pour obtenir une fraction plus légère (C2-, C3-) et une fraction plus lourde (C3+, C4+), pour former le flux d'alimentation de déméthanisation (E) en combinant au moins une partie de la fraction plus légère (C2-, C3-) et au moins une partie du second flux de produits (D), et effectuer l'élimination de l'oxygène (24) en aval de la combinaison, dans laquelle l'installation est conçue pour amener au moins une partie du premier flux de produits (B) à la combinaison sans hydrogénation préalable de l'acétylène ou avec une hydrogénation seulement partielle de l'acétylène, et pour réaliser comprenant l'élimination de l'oxygène (24) en tant qu'élimination de l'acétylène.

11. Installation selon la revendication 10, laquelle est conçue pour la réalisation d'un procédé selon l'une des revendications 1 à 9.
